(19)
Europäisches
Patentamt

European
Patent Office

Office européen
des brevets

(11) **EP 3 575 847 A1**

(12) **EUROPEAN PATENT APPLICATION**
published in accordance with Art. 153(4) EPC

(43) Date of publication:
**04.12.2019 Bulletin 2019/49**

(21) Application number: **17893584.7**

(22) Date of filing: **27.12.2017**

(51) Int Cl.:
*G02B 21/26* (2006.01)          *A61B 90/25* (2016.01)
*G02B 21/00* (2006.01)

(86) International application number:
**PCT/JP2017/046822**

(87) International publication number:
**WO 2018/139156 (02.08.2018 Gazette 2018/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD TN**

(30) Priority: **26.01.2017 JP 2017011831**

(71) Applicant: **Sony Olympus Medical Solutions Inc.
Tokyo 192-0904 (JP)**

(72) Inventors:
• **SAIJO, Hiroki
Hachioji-shi
Tokyo 192-0904 (JP)**
• **YAMAOKA, Nobusuke
Hachioji-shi
Tokyo 192-0904 (JP)**

(74) Representative: **D Young & Co LLP
120 Holborn
London EC1N 2DY (GB)**

(54) **MEDICAL OBSERVATION DEVICE AND CONTROL METHOD**

(57)    Provided is a medical observation apparatus, including: a control unit that controls a zoom of an imaging device based on a distance between the imaging device supported by an arm in which a plurality of links are connected to each other through a joint portion, and an observation target, in which the control unit controls the zoom such that an index value relevant to an actual field of view of the imaging device approximates to a target value that is set.

FIG.5

EP 3 575 847 A1

**Description**

Technical Field

The present disclosure relates to a medical observation apparatus and a control method.

Background Art

Recently, in a medical field, there is a case where a medical observation apparatus capable of performing magnification observation with respect to an observation target such as an affected area, is used in order to support a surgery. Examples of the medical observation apparatus include a medical observation apparatus including an optical microscope, and a medical observation apparatus including an imaging device that functions as an electronic imaging microscope. Hereinafter, the medical observation apparatus including the optical microscope indicates an "optical medical observation apparatus". In addition, hereinafter, there is a case where the medical observation apparatus including the imaging device is referred to as an "electronic imaging medical observation apparatus" or is simply referred to as a "medical observation apparatus".

In the electronic imaging medical observation apparatus, an image quality higher than or equal to that of the optical medical observation apparatus is obtained according to a high image quality of an imaging device, a high image quality of a display device on which a captured image is displayed, or the like. In addition, it is not necessary that a user using the electronic imaging medical observation apparatus (for example, a medical profession such as a surgery operator or an assistant of the surgery operator) peer into an eyepiece configuring the optical microscope, as with the case of using the optical medical observation apparatus, and thus, it is possible to more freely move the position of the imaging device.

In such a circumstance, a technology relevant to the electronic imaging medical observation apparatus has been developed. Examples of the technology described above, include a technology described in Patent Literature 1 described below.

Citation List

Patent Literature

Patent Literature 1: JP 2016-179168 A

Disclosure of Invention

Technical Problem

As described above, the user using the electronic imaging medical observation apparatus, is capable of more freely moving the position of the imaging device. For this reason, the electronic imaging medical observation apparatus is used, and thus, there is an advantage that it is possible to more flexibly support a surgery by moving the position of the imaging device.

However, the position of the imaging device can be more freely moved, and thus, a position relationship between the imaging device and the observation target becomes fluid. In addition, in a case where the position relationship between the imaging device and the observation target is changed, the size of the observation target in the captured image captured by the imaging device may be considerably changed. For this reason, when the electronic imaging medical observation apparatus is used, there is "a case where it is necessary for the user to determine the position of the imaging device, and then, to manually adjust a zoom of the imaging device in order to set the size of the observation target in the captured image to be a more suitable size". Then, the fact that it is necessary for the user to manually adjust the zoom of the imaging device every time when the position relationship between the imaging device and the observation target is changed, leads to a decrease in the convenience of the user.

In the present disclosure, new and improved medical observation apparatus and control method, in which convenience can be improved, are proposed.

Solution to Problem

According to the present disclosure, there is provided a medical observation apparatus, including a control unit that controls a zoom of an imaging device based on a distance between: the imaging device supported by an arm in which a plurality of links are connected to each other through a joint portion; and an observation target, wherein the control unit controls the zoom such that an index value relevant to an actual field of view of the imaging device approximates to a target value that has been set.

Moreover, according to the present disclosure, there is provided a control method executed by a medical observation apparatus, the method including a step of controlling a zoom of an imaging device based on a distance between: the imaging device supported by an arm in which a plurality of links are connected to each other through a joint portion; and an observation target, wherein in the step of controlling the zoom, the zoom is controlled such that an index value relevant to an actual field of view of the imaging device approximates to a target value that has been set.

Advantageous Effects of Invention

According to the present disclosure, it is possible to improve convenience.

Furthermore, the effects described above are not necessarily limited, and any one of the effects described herein or other effects that can be grasped from the description herein may be obtained along with the effects described above or instead of the effects described above.

Brief Description of Drawings

FIG. 1 is an explanatory diagram illustrating an example of a configuration of a medical observation system according to this embodiment.
FIG. 2 is an explanatory diagram illustrating an example of a configuration of an imaging device provided in the medical observation apparatus according to this embodiment.
FIG. 3 is a functional block diagram illustrating an example of the configuration of the medical observation apparatus according to this embodiment.
FIG. 4 is an explanatory diagram for describing an example of zoom control of a control method according to a first embodiment.
FIG. 5 is an explanatory diagram for describing an example of processing of the control method according to the first embodiment.
FIG. 6 is an explanatory diagram illustrating an example of a state transition that is realized by the processing of the control method according to the first embodiment.
FIG. 7 is a flowchart illustrating an example of the processing of the control method according to the first embodiment.
FIG. 8 is an explanatory diagram for describing an example of processing of a control method according to a second embodiment.
FIG. 9 is an explanatory diagram illustrating an example of a state transition that is realized by the processing of the control method according to the second embodiment.
FIG. 10 is a flowchart illustrating an example of the processing of the control method according to the second embodiment.
FIG. 11 is an explanatory diagram for describing a case where processing of a control method according to a third embodiment is not performed.
FIG. 12 is an explanatory diagram for describing the case where the processing of the control method according to the third embodiment is not performed.
FIG. 13 is a flowchart illustrating an example of the processing of the control method according to the third embodiment.

Best Mode for Carrying Out the Invention

Hereinafter, preferred embodiments of the present disclosure will be described in detail with reference to the accompanying drawings. Furthermore, herein and in the drawings, the same reference numerals will be applied to constituents having substantially the same functional configuration, and thus, the repeated description will be omitted.

In addition, hereinafter, the description will be made in the following order.

1. Medical Observation System according to This Embodiment and Control Method according to This Embodiment

[1] Configuration of Medical Observation System

[1-1] Display Device
[1-2] Medical Observation Apparatus

[2] Processing of Control Method according to This Embodiment

[2-1] Processing of Control Method according to First Embodiment
[2-2] Processing of Control Method according to Second Embodiment
[2-3] Processing of Control Method according to Third Embodiment
[2-4] Processing of Control Method according to Fourth Embodiment

2. Program according to This Embodiment

Medical Observation System according to This Embodiment and Control Method according to This Embodiment
Hereinafter, a control method according to this embodiment will be described while describing an example of a medical observation system according to this embodiment described.

[1] Configuration of Medical Observation System

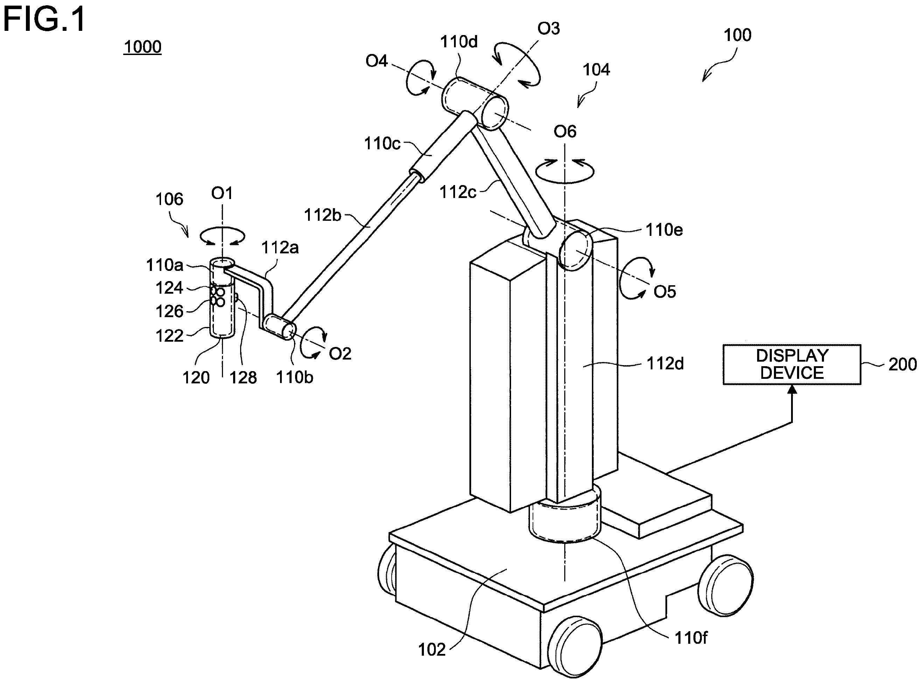

FIG. 1 is an explanatory diagram illustrating an example of a configuration of a medical observation system 1000 according to this embodiment. The medical observation system 1000, for example, includes a medical observation apparatus 100 and a display device 200.
Furthermore, the medical observation system according to this embodiment is not limited to the example illustrated in FIG. 1.
For example, the medical observation system according to this embodiment, may further include a control device (not illustrated) controlling various motions of the medical observation apparatus 100. In the medical observation system 1000 illustrated in FIG. 1, as described below, an example is illustrated in which the medical observation apparatus 100 includes a control unit (described below) performing processing of a control method according to this embodiment, and thus, the medical observation apparatus 100 has a function of the control device (not illustrated).
Examples of the control device (not illustrated) include an arbitrary device that is capable of performing the processing of the control method according to this embodiment, such as a "medical controller" or a "computer such as a server". In addition, the control device (not illustrated), for example, may be an integrated circuit (IC) that can be built in the device as described above.
In addition, the medical observation system according to this embodiment, may include one or both of the medical observation apparatus 100 and the display device 200 in the plural. In a case where there are a plurality of medical observation apparatuses 100, the processing of the control method in the medical observation apparatus 100, described below, is performed in each of the medical observation apparatuses 100. In addition, in a case where the medical observation system according to this embodiment includes a plurality of medical observation apparatuses 100 and a plurality of display devices 200, the medical observation apparatus 100 and the display device 200 may be associated with each other on a one-to-one basis, or the plurality of medical observation apparatuses 100 may be associated with one display device 200. In a case where the plurality of medical observation apparatuses 100 are associated with one display device 200, in the display device 200, for example, a switching operation or the like is performed, and thus, which captured image captured by which medical observation apparatus 100 is displayed on a display screen, is switched. Further, in a case where the medical observation system according to this embodiment includes a plurality of display devices 200, the captured image captured by the medical observation apparatus 100 is displayed on each of the display screens of the plurality of display devices 200.

[1-1] Display Device 200

The display device 200 is a display unit of the medical observation system 1000, and corresponds to an external display device as seen from the medical observation apparatus 100. The display device 200, for example, displays various images such as the captured image (a moving image or a plurality of still images, the same hereinafter) captured by the medical observation apparatus 100, or an image according to a user interface (UI), on the display screen. In addition, the display device 200 may have a configuration in which 3D display can be performed. The display of the display device 200, for example, is controlled by the medical observation apparatus 100 or the control device (not illustrated).
In the medical observation system 1000, the display device 200, for example, is provided in an arbitrary location visible to a person involved in a surgery, such as a surgery operator, in a surgery room, such as a wall surface, a ceiling, of a floor surface of a surgery room. Examples of the display device 200 include a liquid crystal display, an organic EL display, or a cathode ray tube (CRT) display. The display device 200, for example, is driven by power supplied from an internal power source of the display device 200, such as a battery, power supplied from a connected external power source, or the like.

Furthermore, the display device 200 is not limited to the example described above.

For example, the display device 200 may be an arbitrary wearable device that is used by being mounted on the body of the surgery operator or the like, such as a head mounted display or an eyewear type device.

[1-2] Medical Observation Apparatus 100

The medical observation apparatus 100 is an electronic imaging medical observation apparatus. For example, in a case where the medical observation apparatus 100 is used in the surgery, the surgery operator (an example of a user of the medical observation apparatus 100) observes a surgical site with reference to the captured image that is captured by the medical observation apparatus 100 and is displayed on the display screen of the display device 200, and performs various treatments such as a procedure according to a surgery method, with respect to the surgical site.

First, an example of a hardware configuration of the medical observation apparatus 100 will be described with reference to FIG. 1.

The medical observation apparatus 100, for example, includes a base 102, an arm 104, and an imaging device 106.

In addition, even though it is not illustrated in FIG. 1, the medical observation apparatus 100, for example, may include one or two or more processors (not illustrated) configured of an arithmetic circuit such as a micro processing unit (MPU), a read only memory (ROM, not illustrated), a random access memory (RAM, not illustrated), a recording medium (not illustrated), and a communication device (not illustrated). The medical observation apparatus 100, for example, is driven by power supplied from an internal power source of the medical observation apparatus 100, such as a battery, power supplied from a connected external power source, or the like.

The processor (not illustrated) functions as a control unit described below. The ROM (not illustrated) stores control data such as a program or an arithmetic parameter used by the processor (not illustrated). The RAM (not illustrated) temporarily stores the program or the like to be executed by the processor (not illustrated).

The recording medium (not illustrated) is a storage unit of the medical observation apparatus 100, and for example, stores various data items such as data of the control method according to this embodiment such as information indicating the target value described below, and various applications. Here, examples of the recording medium (not illustrated) include a magnetic recording medium such as a hard disk, a non-volatile memory such as flash memory, and the like. In addition, the recording medium (not illustrated) may be detachable from the medical observation apparatus 100.

The communication device (not illustrated) is a communication unit of the medical observation apparatus 100, and has a function of performing communication with respect to an external device such as the display device 200, in a wireless or wired manner. Here, examples of the communication device (not illustrated) include an IEEE802.15.1 port and a transmitting/receiving circuit (wireless communication), an IEEE802.11 port and a transmitting/receiving circuit (wireless communication), a communication antenna and a radio frequency (RF) circuit (wireless communication), or a local area network (LAN) terminal and a transmitting/receiving circuit (wired communication), and the like.

[1-2-1] Base 102

The base 102 is a pedestal of the medical observation apparatus 100, and one end of the arm 104 is connected to the base 102, and thus, the arm 104 and the imaging device 106 are supported by the base 102.

In addition, for example, a caster is provided in the base 102, and the medical observation apparatus 100 is in contact with the floor surface through the caster. The caster is provided, and thus, the medical observation apparatus 100 can be easily moved on the floor surface by the caster.

[1-2-2] Arm 104

In the arm 104, a plurality of links are connected to each other through a joint portion.

In addition, the arm 104 supports the imaging device 106. The imaging device 106 supported by the arm 104 can be three-dimensionally moved, and the position and the posture of the imaging device 106 after being moved, are retained by the arm 104.

More specifically, the arm 104, for example, includes a plurality of joint portions 110a, 110b, 110c, 110d, 110e, and 110f, and a plurality of links 112a, 112b, 112c, and 112d pivotably connected to each other through the joint portions 110a, 110b, 110c, 110d, and 110e. That is, in the medical observation apparatus 100 illustrated in FIG. 1, six freedom degrees relevant to the movement of the imaging device 106 are realized by six rotation axes (a first axis O1, a second axis O2, a third axis O3, a fourth axis O4, a fifth axis O5, and a sixth axis O6) corresponding to six joint portions 110a, 110b, 110c, 110d, 110e, and 110f configuring the arm 104.

The link 112a is an approximately L-shaped member, and each of the links 112b, 112c, and 112d is an approximately rod-like member. One end of the link 112d is connected to the base 102 through the joint portion 110f, and the other end of the link 112d is connected to one end of the link 112c through the joint portion 110e. In addition, the other end of the link 112c is connected to one end of the link 112b through the joint portions 110d and 110c. In addition, the other end of the link 112b is connected to one end of the link 112a through the joint portion 110b, and the other end of the link 112a is connected to the imaging device 106 through the joint portion 110a.

As illustrated in FIG. 1, the ends of each of the plurality of links 112a, 112b, 112c, 112d are pivotably connected to each other through the joint portions 110a, 110b, 110c, 110d, and 110e, with the base 102 as a fulcrum, and thus, the arm 104 in the shape of an arm that is stretched from the base 102, is configured.

An actuator (not illustrated) is provided in each of the joint portions 110a, 110b, 110c, 110d, and 110e, and each of the joint portions 110a, 110b, 110c, 110d, and 110e is rotated by the corresponding rotation axis according to the driving of the actuator (not illustrated). The driving of the actuator (not illustrated), for example, is controlled by the processor functioning as the control unit described below, or an external control device (not illustrated).

Each of the joint portions 110a, 110b, 110c, 110d, and 110e is rotated by the corresponding rotation axis according to the driving of the actuator (not illustrated), and thus, for example, the motion of the arm 104, such as stretching or shrinking (folding) the arm 104, is realized.

As an example, the rotation around the first axis O1 is controlled, and thus, the rotation of the imaging device 106 around an optical axis is controlled. In addition, the rotation around the second axis O2 or the rotation around the third axis O3 are respectively controlled, and thus, the direction of the optical axis of the imaging device 106 with respect to a horizontal surface, is controlled. That is, it can be described that the first axis O1, the second axis O2, and the third axis O3 on a side opposite to the tip end side of the arm 104 (that is, a side opposite to a side to be connected to the base 102), are the rotation axis that mainly controls the posture of the imaging device 106 (the direction of the optical axis). In the medical observation apparatus 100, for example, the rotation of the joint portions 110a, 110b, and 110c corresponding to the first axis O1, the second axis O2, and the third axis O3, is controlled, and thus, the posture of the imaging device 106 is mainly controlled.

In addition, as another example, the rotation around the fourth axis O4, the rotation around the fifth axis O5, and the rotation around the sixth axis O6 are controlled, and thus, a three-dimensional position of the imaging device 106 is controlled. In the medical observation apparatus 100, for example, the rotation of the joint portions 110d, 110e, and 110f corresponding to the fourth axis O4, the fifth axis O5, and the sixth axis O6 on a root side of the arm 104 (that is, a side to be connected to the base 102), is controlled, and thus, the position of the imaging device 106 is mainly controlled.

[1-2-3] Imaging Device 106

The imaging device 106 is supported by the arm 104, and for example, captures an observation target such as a surgical site of a patient. The capturing of the imaging device 106, for example, is controlled by the processor functioning as the control unit described below, or the external control device (not illustrated).

The imaging device 106, for example, has a configuration corresponding to that of an electronic imaging microscope.

More specifically, the imaging device 106, for example, includes an imaging member 120, and a tubular member 122 having an approximately cylindrical shape, and the imaging member 120 is provided in the tubular member 122.

The imaging member 120, for example, includes an optical system provided with an optical element such as one or two or more lenses of an objective lens, a zoom lens, and a focus lens, and a mirror, and an imaging element capturing an image of the observation target by light passing through the optical system.

For example, cover glass for protecting the imaging member 120 is provided on an opening surface of the tubular member 122 on a lower end (in FIG. 1, an end on a lower side).

In addition, for example, a light source is provided in the tubular member 122, and a subject is irradiated with illumination light from the light source through the cover glass at the time of capturing. Reflection light (observation light) from the subject irradiated with the illumination light, is incident on the imaging member 120 through the cover glass, and thus, an image signal indicating the subject (an image signal indicating the captured image) is obtained by the imaging member 120.

A configuration used in various known electronic imaging microscope units, can be applied as the imaging member 120.

As an example, the imaging member 120, for example, includes the optical system, and an image sensor using a plurality of imaging elements such as a complementary metal oxide semiconductor (CMOS) or a charge coupled device (CCD). The imaging member 120 may include a pair of imaging elements, that is, may function as a so-called stereo camera. The imaging member 120 has one or two or more functions such as an auto focus (AF) function including at least a zoom function (one or both of an optical zoom function and an electronic zoom function), generally provided in the electronic imaging microscope unit.

In addition, the imaging member 120, for example, may have a configuration in which capturing can be performed with a so-called high resolution such as 4 K or 8 K. The imaging member 120 is configured such that the capturing can be performed with a high resolution, and thus, it is possible to display an image on the display device 200, for example, including the display screen of a large screen of greater than or equal to 50 inches, while ensuring a predetermined resolution (for example, full HD image quality or the like), and therefore, the visibility of the surgery operator looking at the display screen, is improved. In addition, the imaging member 120 is configured such that the capturing can be performed with a high resolution, and thus, even in a case where the captured image is magnified by the electronic zoom function and is displayed on the display screen of the display device 200, it is possible to ensure a predetermined resolution. Further, in a case where a predetermined resolution is ensured by using the electronic zoom function, it is possible to suppress the performance of the optical zoom function of the imaging device 106, and thus, it is possible to more simplify the optical system of the imaging device 106, and to configure the imaging device 106 to have a smaller size.

For example, various operating devices for controlling the motion of the imaging device 106 are provided in the imaging device 106. For example, in FIG. 1, a zoom switch 124, a focus switch 126, and a motion mode change switch 128 are provided in the imaging device 106. Furthermore, it is obvious that the position and the shape in which the zoom switch 124, the focus switch 126, and the motion mode change switch 128 are provided, are not limited to the example illustrated in FIG. 1.

The zoom switch 124 and the focus switch 126 are an example of an operating device for adjusting an imaging condition of the imaging device 106. An operation with respect to the zoom switch 124 is performed, and thus, a zoom magnification is adjusted, and a zoom is adjusted. In addition, an operation with respect to the focus switch 126 is performed, and thus, a focal distance is adjusted, and a focus is adjusted. The operation with respect to the zoom switch 124 corresponds to an operation with respect to the zoom described below.

The motion mode change switch 128 is an example of an operating device for changing a motion mode of the arm 104 of the imaging device 106. An operation with respect to the motion mode change switch 128 is performed, and thus, the motion mode of the arm 104 is changed. Examples of the motion mode of the arm 104 include a fixed mode and a free mode.

Here, the fixed mode according to this embodiment, for example, is a motion mode in which the rotation of each of the rotation axes provided in the arm 104 is regulated by a brake, and thus, the position and the posture of the imaging device 106 are fixed.

In addition, the free mode according to this embodiment, is a motion mode in which the brake is released, and thus, each of the rotation axes provided in the arm 104 can be freely rotated. For example, in the free mode, the position and the posture of the imaging device 106 can be adjusted by a direct operation of the surgery operator. Here, the direct operation according to this embodiment, for example, indicates an operation in which the surgery operator grips the imaging device 106 with hands, and directly moves the imaging device 106.

Examples of the operation with respect to the motion mode change switch 128, include an operation of pressing the motion mode change switch 128. For example, the motion mode of the arm 104 becomes the free mode while the surgery operator presses the motion mode change switch 128, and when the surgery operator does not press the motion mode change switch 128, the motion mode of the arm 104 becomes the fixed mode.

Further, for example, a distance sensor (not illustrated in FIG. 1) measuring a distance between the imaging device 106 and the observation target, is provided in the imaging device 106. Examples of the distance sensor (not illustrated in FIG. 1) include an arbitrary type sensor capable of measuring the distance between the imaging device 106 and the observation target, such as a distance sensor using light of a predetermined wavelength, such as an infrared ray, a distance sensor using a sound wave such as an ultrasound wave. Hereinafter, there is a case where the distance between the imaging device 106 and the observation target is referred to as a "distance with respect to the observation target".

A detection result of the distance sensor (not illustrated in FIG. 1), for example, is transferred to the processor functioning as the control unit described below, and in the processor, the distance with respect to the observation target which is specified based on the detection result of the distance sensor (not illustrated in FIG. 1), is used in the processing of the control method according to this embodiment described below. Here, the detection result of the distance sensor (not illustrated in FIG. 1) may indicate the distance with respect to the observation target, or the distance may be calculated by the processor functioning as the control unit described below, based on the detection result of the distance sensor (not illustrated in FIG. 1).

The image signal (image data) generated by capturing of the imaging device 106, for example, is subjected to image processing in the processor functioning as the control unit described below. Examples of the image processing according to this embodiment include one or two or more pieces of processing of various pieces of processing such as gamma correction, the adjustment of a white balance, the magnification or reduction of an image according to the electronic zoom function, or inter-pixel correction. Furthermore, in a case where the medical observation system according to this embodiment includes the control device (not illustrated) controlling various motions of the medical observation apparatus 100, the image processing according to this embodiment may be performed in the control device (not illustrated).

The medical observation apparatus 100, for example, transmits a display control signal, and an image signal subjected to the image processing as described above, to the display device 200. The display control signal and the image signal are transmitted to the display device 200, and thus, the captured image obtained by capturing the observation target (for example, the captured image in which the surgical site is captured) is magnified or reduced at a desired magnification according to one or both of the optical zoom function and the electronic zoom function, and is displayed on the display screen of the display device 200.

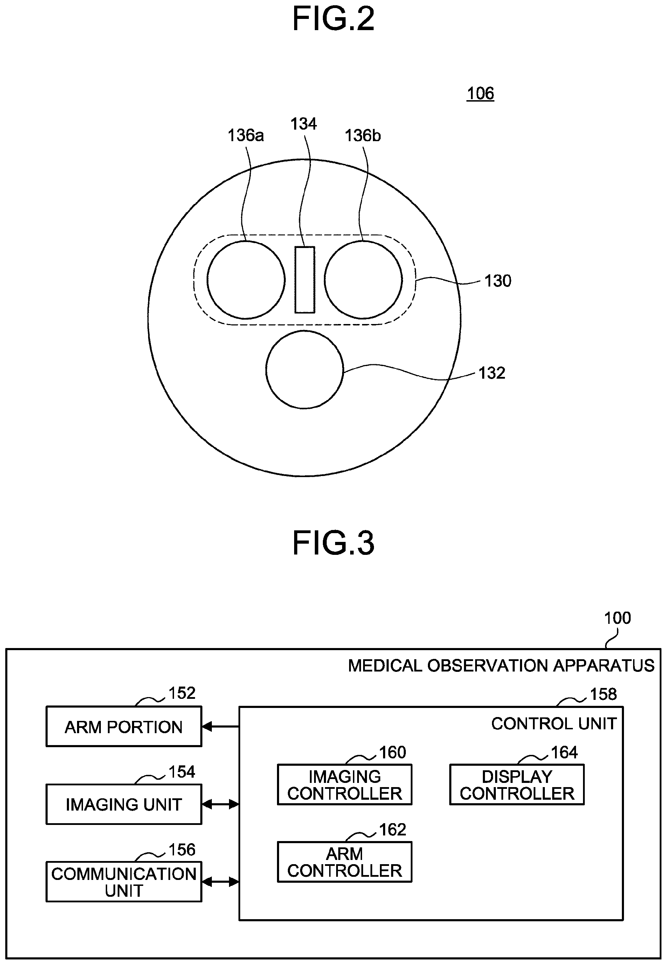

FIG. 2 is an explanatory diagram illustrating an example of the configuration of the imaging device 106 of the medical observation apparatus 100 according to this embodiment, and corresponds to a diagram of the imaging device 106 illustrated in FIG. 1 as seen from a lower direction of FIG. 1. The imaging device 106, for example, includes a first imaging device 130, a second imaging device 132, and a distance sensor 134.

The first imaging device 130 is an imaging device for capturing the observation target such as a surgical site. In FIG. 2, the first imaging device 130 includes an imaging device 136a capturing a captured image for right eye, and an imaging device 136b capturing a captured image for left eye, and functions as a stereo camera. Hereinafter, there is a case where an imaging range of the first imaging device 130 is referred to as a "first viewing region".

The second imaging device 132 is an imaging device for capturing a region including the observation target such as a surgical site, and the circumference of the observation target. Hereinafter, there is a case where an imaging range of the second imaging device 132 is referred to as a "second viewing region". That is, the second viewing region is a region that includes the first viewing region, and is larger than the first viewing region.

In order to capture the second viewing region, an imaging device having an angle of view wider than that of the first imaging device 130, is used as the second imaging device 132. As an example, in the second imaging device 132, a wide-angle lens or a fish-eye lens is provided as a lens of the optical system. Here, the wide-angle lens is a lens having an angle of view wider than that of at least the lens of the optical system of the first imaging device 130. In addition, the fish-eye lens, for example, is a lens having an angle of view (a nominal diagonal angle of view) that is close or greater than or equal to 170[°].

The distance sensor 134 is a sensor measuring the distance with respect to the observation target. As described above, examples of the distance sensor 134 include a distance sensor using light of a predetermined wavelength, such as an infrared ray, a distance sensor using a sound wave such as an ultrasound wave, or the like.

The imaging device 106, for example, has a configuration illustrated in FIG. 2. Furthermore, the configuration of the imaging device 106 is not limited to the example illustrated in FIG. 2. For example, the imaging device 106 may not include the second imaging device 132. In addition, the first imaging device 130 of the imaging device 106 may not function as the stereo camera. Further, a position in which the distance sensor 134 is provided in the imaging device 106, is not limited to the example illustrated in FIG. 2, and the imaging device 106 may not include the distance sensor 134.

The medical observation apparatus 100, for example, has the hardware configuration described with reference to FIG. 1.

Furthermore, the hardware configuration of the medical observation apparatus according to this embodiment, is not limited to the configuration described with reference to FIG. 1.

For example, the medical observation apparatus according to this embodiment does not include the base 102, and may have a configuration in which the arm 104 is directly attached to the ceiling, the wall surface, or the like of the surgery room or the like. For example, in a case where the arm 104 is attached to the ceiling, the medical observation apparatus according to this embodiment has a configuration in which the arm 104 is suspended from the ceiling.

In addition, in FIG. 1, an example is illustrated in which the arm 104 is configured such that six freedom degrees are realized with respect to the driving of the imaging device 106, but the configuration of the arm 104 is not limited to the configuration in which the freedom degree with respect to the driving of the imaging device 106 is six freedom degrees. For example, the arm 104 may be configured such that the imaging device 106 can be suitably moved according to the application, and the number of joint portions and links, the arrangement of the joint portions and the links, the direction of a driving axis of the joint portion, or the like can be suitably set such that the arm 104 has a desired freedom degree.

In addition, in FIG. 1, an example is illustrated in which various operating devices for controlling the motion of the imaging device 106, are provided in the imaging device 106, but a part or all of the operating devices illustrated in FIG. 1 may not be provided in the imaging device 106. As an example, various operating devices for controlling the motion of the imaging device 106, may be provided in other portions in addition to the imaging device 106 configuring the medical observation apparatus according to this embodiment. In addition, as another example, various operating devices for controlling the motion of the imaging device 106 may be an external operating device such as a remote controller or a foot switch.

In addition, in FIG. 1, for the sake of simplicity, the tubular member 122 of the imaging device 106 is illustrated as a member having a simple cylindrical shape, but a grip member that is gripped by the surgery operator, can be provided in the tubular member 122. Examples of the grip member include a member having a handle structure that can be gripped by the surgery operator, and a member having an arbitrary shape that can be gripped by the surgery operator.

Further, in the above description, an example has been described in which the distance sensor 134 is provided in the imaging device 106, but in the medical observation apparatus 100, the distance sensor 134 is provided in an arbitrary position where the distance between the imaging device 106 and the observation target can be obtained.

Next, the medical observation apparatus 100 illustrated in FIG. 1, will be described by using a function block. FIG. 3 is a functional block diagram illustrating an example of the configuration of the medical observation apparatus 100 according to this embodiment.

The medical observation apparatus 100, for example, includes an arm portion 152, an imaging unit 154, a communication unit 156, and a control unit 158.

The arm portion 152 includes the arm 104, and supports the imaging device 106 configuring the imaging unit 154.

The imaging unit 154 includes the imaging device 106, and captures the observation target. The capturing of the imaging unit 154, for example, is controlled by the control unit 158.

The communication unit 156 is the communication unit of the medical observation apparatus 100, has a function of performing communication with respect to an external device such as the display device 200, in a wireless or wired manner. The communication unit 156, for example, includes the communication device (not illustrated) described above. The communication of the communication unit 156, for example, is controlled by the control unit 158.

The control unit 158, for example, includes the processor (not illustrated) described above, and has a function of controlling the entire medical observation apparatus 100. In addition, the control unit 158 has a function of initiatively performing the processing of the control method described below.

More specifically, the control unit 158, for example, includes an imaging controller 160, an arm controller 162, and a display controller 164.

The imaging controller 160 controls the imaging device 106 configuring the imaging unit 154. Examples of the control of the imaging device 106 include the control of one or two or more functions such as the control of the AF function including the control of at least the zoom function (one or both of the optical zoom function and the electronic zoom function), generally provided in the electronic imaging microscope unit.

In addition, the imaging controller 160 performs the processing of the control method described below.

The arm controller 162 controls the driving of the arm 104 configuring the arm portion 152. Examples of the control of the driving of the arm 104 include "applying a control signal of controlling the driving to the actuator (not illustrated) corresponding to each of the joint portions 110a, 110b, 110c, 110d, and 110e" and the like.

The display controller 164, for example, transfers the display control signal and the image signal to the communication device (not illustrated) configuring the communication unit 156, and transmits the display control signal and the image signal to the display device 200, and thus, controls the display of the display device 200. Furthermore, the control of the communication of the communication unit 156 may be performed by a communication control unit (not illustrated) configuring the control unit 158.

As illustrated in FIG. 2, there is a case where the imaging device 106 has a "configuration including the first imaging device 130 capturing the first viewing region, and the second imaging device 132 capturing the second viewing region".

In a case where the imaging device 106 has a configuration as illustrated in FIG. 2, the display controller 164, for example, simultaneously displays the captured image captured by the first imaging device 130, and the captured image captured by the second imaging device 132, on the display screen of the display device 200 (an example of the display screen of the same display device). Examples of the simultaneous display include "displaying the captured image of the second imaging device 132 on a display area of a part of the display screen on which the captured image of the first imaging device 130 is displayed". The display controller 164, for example, transmits the display control signal of performing display in an arbitrary method in which a plurality of images such as a picture in picture (PIP) or a picture on picture (POP), can be displayed on one screen, to the display device 200.

In addition, in a case where the imaging device 106 has a configuration as illustrated in FIG. 2, the display controller 164 may respectively display the captured image captured by the first imaging device 130, and the captured image captured by the second imaging device 132, on display screens of different display devices. The display controller 164 transmits the display control signal and the image signal corresponding to the first imaging device 130, and the display control signal and the image signal corresponding to the second imaging device 132, to different display devices.

The control unit 158, for example, includes the imaging controller 160, and thus, has a function of initiatively performing the processing of the control method according to this embodiment. In addition, the control unit 158, for example, includes the arm controller 162 and the display controller 164, and thus, has a function of controlling the entire medical observation apparatus 100.

Furthermore, the configuration of the control unit 158 is not limited to the example illustrated in FIG. 3. For example, the control unit 158 is capable of having an arbitrary configuration according to a separation method of the functions of the medical observation apparatus 100, such as a configuration according to a separation method of the processing of the control method according to this embodiment.

The medical observation apparatus 100, for example, performs the processing of the control method according to this embodiment described below, according to the configuration illustrated in FIG. 3.

Furthermore, the configuration of the medical observation apparatus according to this embodiment, is not limited to the configuration illustrated in FIG. 3.

For example, the medical observation apparatus according to this embodiment is capable of including one or two or more of the imaging controller 160, the arm controller 162, and the display controller 164, illustrated in FIG. 3, separately from the control unit 158 (for example, realizing one or two or more of the imaging controller 160, the arm controller 162, and the display controller 164 in another processing circuit).

In addition, in the medical observation apparatus according to this embodiment, the configuration for realizing the processing of the control method according to this embodiment, is not limited to the configuration illustrated in FIG. 3, and can be the configuration according to the separation method of the processing of the control method according to this embodiment.

In addition, for example, in a case where the communication with respect to the external device is performed through an external communication device having the same function and the same configuration as those of the communication unit 156, the medical observation apparatus according to this embodiment may not include the communication unit 156.

In addition, in a case where the medical observation system according to this embodiment has a configuration including the control device (not illustrated), and the medical observation apparatus according to this embodiment is controlled by the control device (not illustrated), the medical observation apparatus according to this embodiment may not include the control unit 158.

Here, the control device (not illustrated), for example, includes the control unit having the same function and the same configuration as those of the control unit 158, and thus, performs the processing of the control method according to this embodiment described below, and controls the motion of each constituent of the medical observation apparatus according to this embodiment, such as the arm portion 152 or the imaging unit 154. The control device (not illustrated) performs communication with respect to the medical observation apparatus according to this embodiment through the provided communication device or the connected external communication device, and thus, controls the motion of each of the constituents of the medical observation apparatus according to this embodiment.

[2] Processing of Control Method according to This Embodiment

Next, the processing of the control method according to this embodiment will be described. Hereinafter, a case where the processing of the control method according to this embodiment is performed by the medical observation apparatus 100 (more specifically, for example, the control unit 158 configuring the medical observation apparatus 100), will be exemplified. Furthermore, as described above, in the medical observation system according to this embodiment, the processing of the control method according to this embodiment may be performed by the control device (not illustrated).

[2-1] Processing of Control Method according to First Embodiment

For example, in a case where the imaging device 106 is supported by the arm 104 as illustrated in FIG. 1, the position of the imaging device 106 can be freely moved.

However, as described above, the position of the imaging device 106 can be freely moved, and thus, a position relationship between the imaging device 106 and the observation target becomes fluid. For this reason, in a case where the position relationship between the imaging device 106 and the observation target is changed, the size of the observation target in the captured image captured by the imaging device 106 may be considerably changed.

In addition, as described above, in a case where when the size of the observation target in the captured image is changed, it is necessary for the user of the medical observation apparatus 100 to manually adjust the zoom of the imaging device 106 in order to set the size of the observation target in the captured image to be a more suitable size, there is a concern that the convenience of the user is impaired. Examples of the user of the medical observation apparatus 100 according to this embodiment include a medical profession such as a surgery operator or an assistant of the surgery operator.

Therefore, the medical observation apparatus 100 controls the zoom of the imaging device 106, based on the distance between the imaging device 106 supported by the arm 104 and the observation target.

More specifically, the medical observation apparatus 100 controls the zoom such that an index value relevant to an actual field of view of the imaging device 106 approximates to a target value (described below) that is set. The index value relevant to the actual field of view of the imaging device 106 according to this embodiment, is a value that becomes an index at the time of controlling the zoom. Examples of the index value relevant to the actual field of view of the imaging device 106 include an arbitrary value that is capable of indicating the actual field of view of the imaging device 106, such as a diameter of the actual field of view of the imaging device 106, a radius of the actual field of view of the imaging device 106, the length of the outer circumference of the actual field of view of the imaging device 106, and an area of the actual field of view of the imaging device 106. Hereinafter, a case where the index value relevant to the actual field of view of the imaging device 106 is the diameter of the actual field of view of the imaging device 106, will be exemplified.

The medical observation apparatus 100 controls the zoom such that the index value approximates to the target value (described below), and thus, the size of the observation target in the captured image captured by the imaging device 106, can be identical before the distance with respect to the observation target is changed and after the distance is changed. That is, the medical observation apparatus 100 controls the zoom such that the size of the observation target in the captured image captured by the imaging device 106 is identical before the distance with respect to the observation target is changed and after the distance is changed. In other words, the medical observation apparatus 100 controls the zoom such that the index value relevant to the actual field of view of the imaging device 106 is identical before the distance with respect to the observation target is changed and after the distance is changed. Here, "the size of the observation target in the captured image being identical before the distance with respect to the observation target is changed and after the distance is changed" indicates that "the size of the observation target before the change is completely coincident with the size after the change" or " a difference in the size is sufficiently small to the extent that a person peering at the captured image feels that the size of the observation target before the change is coincident with the size after the change". In addition, "the index value relevant to the actual field of view of the imaging device 106 being identical before the distance with respect to the observation target is changed and after the distance is changed" indicates that "the index value relevant to the actual field of view of the imaging device 106 before the change is completely coincident with the index value after the change" or "a difference in the index value relevant to the actual field of view of the imaging device 106 is sufficiently small to the extent that a person peering at the captured image feels that the size of the observation target before the change is coincident with the size after the change". Each of the upper limit value of a sufficiently small difference in the size, and the upper limit value of a sufficiently small difference in the index value relevant to the actual field of view of the imaging device 106, for example, can be experimentally determined by quantitatively evaluating the result of a "qualitative experiment of allowing a test subject to compare images, and of obtaining a response of whether or not the test subject feels that the compared images are coincident with each other".

The distance between the imaging device 106 and the observation target, for example, is detected by the distance sensor 134, and the medical observation apparatus 100 controls the zoom based on the distance with respect to the observation target acquired from the distance sensor 134. In addition, the medical observation apparatus 100, for example, may assume the distance with respect to the observation target based on a focus result according to the control of the AF function, and may control the zoom based on the distance with respect to the observation target acquired by the assumption.

Here, the medical observation apparatus 100, for example, controls the zoom based on the distance with respect to the observation target detected at a certain time point after the distance with respect to the observation target is changed. The medical observation apparatus 100 controls the zoom based on the distance with respect to the observation target every time when the distance with respect to the observation target is acquired.

In addition, the medical observation apparatus 100, for example, may filter the distance at a plurality of time points after the distance with respect to the observation target is changed, and may control the zoom based on the filtered distance. The medical observation apparatus 100, for example, performs filter processing using an arbitrary filter such as a smoothing filter, obtains the distance with respect to one observation target, from the distances with respect to the plurality of observation targets respectively corresponding to the plurality of time points, and controls the zoom based on the distance with respect to the observation target which is obtained.

Examples of the control of the zoom according to this embodiment control include the control of the zoom magnification of the imaging device 106 based on the distance with respect to the observation target.

Here, the upper limit value and the lower limit value according to the zoom performance of the imaging device 106 exist in the zoom magnification of the imaging device 106. For this reason, the medical observation apparatus 100 controls the zoom within a range of the upper limit value of the zoom magnification and the lower limit value of the zoom magnification of the imaging device 106.

Furthermore, in a "case where the zoom magnification exceeds the zoom performance of the imaging device 106 when the zoom is controlled such that the diameter of the actual field of view of the imaging device 106 (an example of the index value relevant to the imaging device 106, the same hereinafter) is identical before the distance with respect to the observation target is changed and after the distance is changed", the medical observation apparatus 100 controls the zoom such that the zoom magnification becomes closer to the upper limit value of the zoom magnification and the lower limit value of the zoom magnification of the imaging device 106.

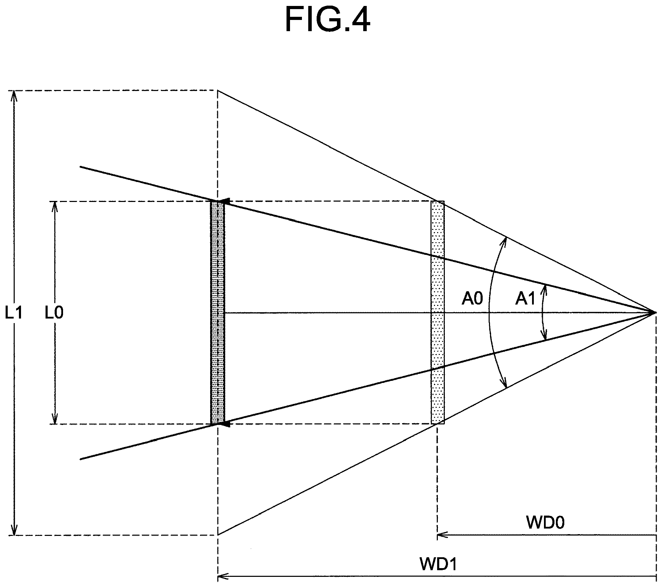

FIG. 4 is an explanatory diagram for describing an example of the control of the zoom of the control method according to the first embodiment. "A0" illustrated in FIG. 4 represents a viewing angle of the imaging device 106 when the zoom magnification is 1.0 times, and "A1" illustrated in FIG. 4 represents the viewing angle of the imaging device 106 after automatic zoom adjustment of the control method according to this embodiment is performed. In addition, "WD0" illustrated in FIG. 4 represents the distance before the distance between the imaging device 106 and the observation target is changed, and "WD1" illustrated in FIG. 4 represents the distance after the distance between the imaging device 106 and the observation target is changed. In addition, "L0" illustrated in FIG. 4 represents the diameter of the actual field of view in a case where the distance between the imaging device 106 and the observation target is the distance WD0 when the zoom magnification is 1.0 times, and "L1" illustrated in FIG. 4 represents the diameter of the actual field of view in a case where the distance between the imaging device 106 and the observation target is the distance WD1 when the zoom magnification is 1.0 times.

Here, the diameter L0 of the actual field of view in a case where the distance between the imaging device 106 and the observation target is the distance WD0, is represented by Equation 1 described below.

$$L0 = 2 \times WD0 \times \tan(A0/2) \ldots \text{(Equation 1)}$$

In addition, the diameter L1 of the actual field of view in a case where the distance between the imaging device 106 and the observation target is the distance WD1, is represented by Equation 2 described below.

$$L1 = 2 \times WD1 \times \tan(A0/2) \ldots \text{(Equation 2)}$$

In addition, a zoom magnification X for setting the same actual field of view in a case where the distance with respect to the observation target is the distance WD0 and in a case where the distance with respect to the observation target is the distance WD1, is represented by Equation 3 described below, from Equation 1 described above and Equation 2 described above.

$$X = L1/L0 = WD1/WD0 \ldots \text{(Equation 3)}$$

Furthermore, the viewing angle of the imaging device 106 in the case of the zoom magnification X represented by Equation 3 described above, that is, the viewing angle A1 of the imaging device 106 after the automatic zoom adjustment, is represented by Equation 4 described below.

$$A1 = 2 \times \operatorname{atan}\{(L0/2)/WD1\} \ldots \text{(Equation 4)}$$

As described with reference to FIG. 4, the medical observation apparatus 100, for example, controls the zoom by obtaining the zoom magnification in which the diameter of the actual field of view is identical before the distance with respect to the observation target is changed and after the distance is changed.

Furthermore, in the example referring to FIG. 4, an example is illustrated in which the zoom magnification before the distance with respect to the observation target is changed, is 1.0 times, but even in a case where the zoom magnification before the distance changed is an arbitrary zoom magnification that can be set by the imaging device 106, the medical observation apparatus 100 is capable of controlling the zoom by obtaining the zoom magnification, as with the example described with reference to FIG. 4.

The processing of the control method according to the first embodiment as described above is performed, and thus, the automatic zoom adjustment based on the distance between the imaging device 106 and the observation target, is realized. Hereinafter, there is a case where the automatic zoom adjustment based on the distance between the imaging device 106 and the observation target according to this embodiment, that is, the automatic zoom adjustment of the control method according to this embodiment, is referred to as "automatic zoom" or "follow-up adjustment". In addition, hereinafter, there is a case where manual zoom adjustment is referred to as "manual zoom".

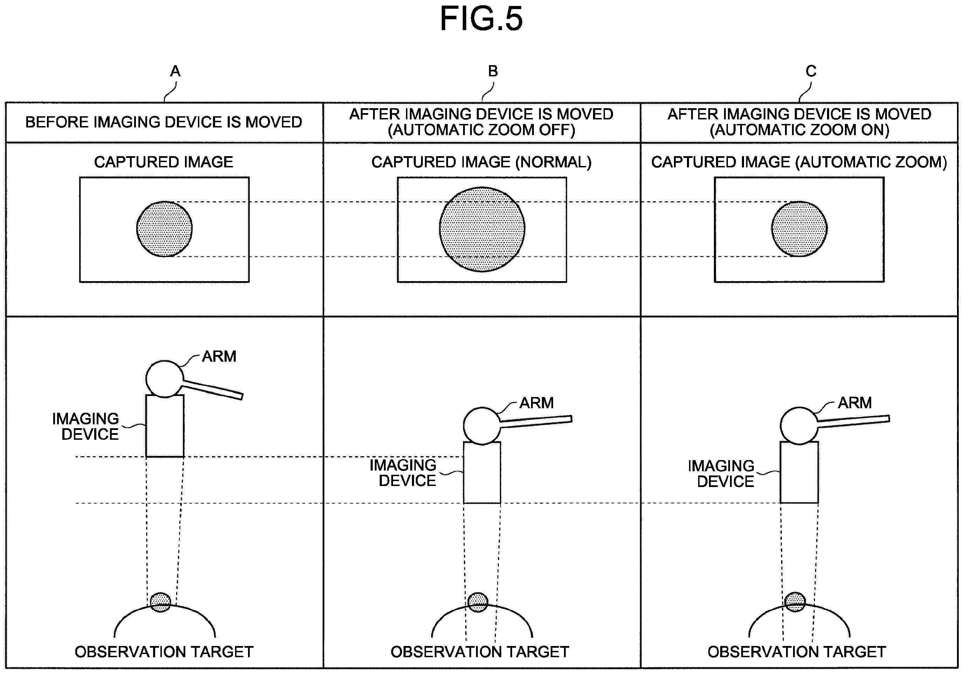

FIG. 5 is an explanatory diagram for describing an example of the processing of the control method according to the first embodiment.

A of FIG. 5 illustrates an example of the captured image before the imaging device 106 is moved, that is, an example of the captured image before the distance with respect to the observation target is changed.

In addition, B of FIG. 5 and C of FIG. 5 respectively illustrate an example of the captured image after the imaging device 106 is moved, that is, an example of the captured image after the after the distance with respect to the observation target is changed. More specifically, B of FIG. 5 illustrates a case where the processing of the control method according to the first embodiment is not performed, that is, an example of the captured image in a state where the automatic zoom is turned off. In addition, C of FIG. 5 illustrates a case where the processing of the control method according to the first embodiment is performed, that is, an example of the captured image in a state where the automatic zoom is turned on.

As illustrated in A of FIG. 5, B of FIG. 5, and C of FIG. 5, it is known that in a case where the automatic zoom is performed, the size of the observation target in the captured image is identical before the distance is changed and after the distance is changed.

Accordingly, the processing of the control method according to the first embodiment is performed, and thus, even in a case where the user of the medical observation apparatus 100 moves the medical observation apparatus 100, it is not necessary to manually adjust the zoom of the imaging device 106. Therefore, the medical observation apparatus 100 performing the processing of the control method according to the first embodiment, is used, and thus, it is possible to improve the convenience.

Hereinafter, the processing of the control method according to the first embodiment will be described in more detail.

The medical observation apparatus 100 controls the zoom based on the distance with respect to the observation target such that the index value relevant to the actual field of view of the imaging device 106 is identical to the target value that is set.

The target value according to this embodiment, for example, is an index value relevant to the actual field of view that is a target of the automatic zoom. As an example, the diameter L0 of actual field of view illustrated in FIG. 4 corresponds to the target value according to this embodiment. In addition, the target value according to this embodiment, for example, may be a value corresponding to the radius of the actual field of view of the imaging device 106, a value corresponding to the length of the outer circumference of the actual field of view of the imaging device 106, a value corresponding to the area of the actual field of view of the imaging device 106, and the like. The medical observation apparatus 100 controls the zoom such that the index value relevant to the actual field of view of the imaging device 106 is identical to the target value, and thus, it is realized that the index value relevant to the actual field of view of the imaging device 106 is identical before the distance with respect to the observation target is changed and after the distance is changed.

The target value according to this embodiment, for example, is set by storing information (data) indicating the target value in the recording medium (not illustrated). In addition, the medical observation apparatus 100, for example, reads out the information (data) indicating the target value from the recording medium (not illustrated), and thus, specifies the target value that is set.

Here, examples of the target value according to this embodiment include a value set with respect to the medical observation apparatus 100.

In a case where the target value is the value set with respect to the medical observation apparatus 100, examples of the information indicating the target value include numerical data indicating the value set with respect to the medical observation apparatus 100. The medical observation apparatus 100 uses the value indicating the information indicating the target value, in the processing, as the target value.

Furthermore, the target value according to this embodiment is not limited to the example described above. For example, the target value according to this embodiment may be a value associated with one or both of the user of the medical observation apparatus and the surgery method in which the medical observation apparatus 100 (or the procedure, the same hereinafter) is used.

In a case where the target value is the value associated with one or both of the user of the medical observation apparatus and the surgery method in which the medical observation apparatus 100 is used, examples of the information indicating the target value include a "table (or a database) in which one or both of information indicating the user of the medical observation apparatus 100 and information indicating the surgery method, and the target value are associated with each other". Hereinafter, there is a case where the table described above is referred to as a "target value table".

Here, examples of the information indicating the user of the medical observation apparatus 100 include arbitrary data that is capable of specifying the user of the medical observation apparatus 100, such as data indicating an ID of the user of the medical observation apparatus 100, or biological information of the user of the medical observation apparatus 100. In addition, examples of the information indicating the surgery method include arbitrary data that is capable of specifying the surgery method (or the procedure), such as data indicating an ID indicating the surgery method (or the procedure), or text data indicating the surgery method (or the procedure).

The medical observation apparatus 100, for example, specifies the target value based on one or both of the information indicating the user and the information indicating the surgery method, and the target value table (an example of the information indicating the target value), acquired by a predetermined operation such as an ID input operation of the user, and uses the specified target value in the processing.

The target value according to this embodiment, for example, is changed based on an operation relevant to the zoom, such as the operation with respect to the zoom switch 124. As described above, the target value is changed based on the operation relevant to the zoom, and thus, the medical observation apparatus 100 is capable of performing the automatic zoom such that the size of the observation target in the captured image after the manual zoom adjustment, is retained.

Accordingly, the medical observation apparatus 100 changes the target value based on the operation relevant to the zoom, adn thus, it is possible to further improve the convenience of the user of the medical observation apparatus 100.

In addition, in a case where the target value is changed based on the operation relevant to the zoom, the medical observation apparatus 100 records the changed target value in the recording medium (not illustrated). The medical observation apparatus 100, for example, may overwrite and update the target value indicated by the information indicating the target value, stored in the recording medium (not illustrated), with the changed target value, or may record information indicating a new target value indicating the changed target value, in the recording medium (not illustrated). In a case where the information indicating the new target value is recorded in the recording medium (not illustrated), for example, information indicating a plurality of target values having different time stamps, is stored in the recording medium (not illustrated).

As described above, the medical observation apparatus 100 records the changed target value in the recording medium (not illustrated), and thus, the target value after the manual zoom adjustment, is stored. In addition, the medical observation apparatus 100 is capable of performing the automatic zoom based on the stored target value after the manual zoom adjustment. Accordingly, for example, the user of the medical observation apparatus 100, performing the manual zoom adjustment in a certain surgery, is capable of receiving the benefit of the automatic zoom reflecting the result of the manual zoom adjustment that is previously performed in another surgery to be performed later, without performing again the manual zoom adjustment.

Therefore, the medical observation apparatus 100 records the changed target value in the recording medium (not illustrated), and thus, it is possible to further improve the convenience of the user of the medical observation apparatus 100.

Furthermore, the processing of the control method according to the first embodiment is not limited to the example described above. The medical observation apparatus 100, for example, is capable of further performing one or two or more pieces of processing of the pieces of processing according to the first example represented in (1) described below to processing according to a fourth example represented in (4) described below.

(1) First Example of Processing of Control Method

In a case where the operation relevant to the zoom is detected, the medical observation apparatus 100 controls the zoom such that the zoom corresponds to the operation relevant to the zoom. That is, in the medical observation apparatus 100, for example, the zoom magnification can be changed according to an operation amount (or the number of times of the operation) of the operation relevant to the zoom such as the operation with respect to the zoom switch 124.

As described above, in the medical observation apparatus 100, the automatic zoom and the manual zoom are performed. In addition, in a case where the automatic zoom and the manual zoom are performed, the operation relevant to the zoom may be performed when the automatic zoom is performed.

Here, it is considered that there are many "cases where the user of the medical observation apparatus 100 magnifies or reduces the captured image displayed on the display screen to a desired size", as a case where the operation relevant to the zoom is performed when the automatic zoom is performed. For this reason, in a case where the operation relevant to the zoom is performed when the automatic zoom is performed, it is considered that the effect of improving the convenience of the user of the medical observation apparatus 100 is higher in a case where the manual zoom is prioritized over the automatic zoom.

Accordingly, in a case where the operation relevant to the zoom is detected when the zoom is controlled based on the distance with respect to the observation target (when the automatic zoom is performed), the medical observation apparatus 100 stops the control of the zoom based on the distance. Then, the medical observation apparatus 100 controls the zoom such that the zoom corresponds to the operation relevant to the zoom.

As described above, in the medical observation apparatus 100, the target value is changed based on the operation relevant to the zoom. Accordingly, the medical observation apparatus 100 performs new automatic zoom that is performed after the automatic zoom is stopped, based on the changed target value.

(2) Second Example of Processing of Control Method

In a case where it is determined that a predetermined operation that is set, is ended, the medical observation apparatus 100 controls the zoom based on the distance with respect to the observation target.

Examples of the predetermined operation according to this embodiment include operations with respect to each of various operating devices for controlling the motion of the imaging device 106, such as the zoom switch 124, the focus switch 126, and the motion mode change switch 128, illustrated in FIG. 1. In addition, the predetermined operation according to this embodiment may be an operation with respect to the external operating device of the medical observation apparatus 100, such as a remote controller or a foot switch.

For example, "when the predetermined operation is detected, and processing corresponding to the predetermined operation is completed" or "when the processing corresponding to the predetermined operation is completed, and then, a new predetermined operation is not detected within a standby time that is set", the medical observation apparatus 100 determines that the end of the predetermined operation is detected.

As described above, in a case where it is determined that the predetermined operation is ended, the automatic zoom is performed, and thus, in the medical observation apparatus 100, the predetermined operation performed by the user of the medical observation apparatus 100 is prioritized. Accordingly, as with a case where the manual zoom is prioritized over the automatic zoom, in the first example of (1) described above, the medical observation apparatus 100 is capable of improving the convenience of the user of the medical observation apparatus 100.

(3) Third Example of Processing of Control Method

In a case where the medical observation apparatus 100 has a pivot motion function, the medical observation apparatus 100 may perform the automatic zoom in conjunction with the pivot motion function.

A pivot motion according to this embodiment is a motion in which the imaging device 106 is moved on the plane of a circular cone having a predetermined point as the apex, in a state of being consistently directed towards the predetermined point on a space (that is, in a state where the optical axis of the imaging device 106 consistently passes through the predetermined point on the space). The pivot motion is performed, and thus, it is realized that the imaging device 106 performs capturing while automatically changing the direction, to follow a specific position (for example, one portion of the surgical site). In addition, the pivot motion of the medical observation apparatus 100 is realized by controlling the motion of the arm 104 supporting the imaging device 106.

In a case where the pivot motion is performed, the imaging device 106, for example, is pivoted around the axis of the circular cone having the predetermined point on the space as the apex, as a pivot axis. The predetermined point on the space described above, is a point indicating the center of the pivot motion, and thus, can also be referred to as a pivot center. In a case where the predetermined point on the space described above is set in the surgical site of the patient, the imaging device 106 is capable of motioning to pivot around the surgical site, in a state where the surgical site is captured. Accordingly, the medical observation apparatus 100 is capable of capturing the surgical site of the patient in which the predetermined point on the space described above is set, from various directions.

Here, in a case where the pivot motion is performed, the distance between the imaging device 106 and the observation target is not constantly retained, and thus, the distance with respect to the observation target is capable of varying. In a case where the pivot motion is performed, the medical observation apparatus 100 controls the zoom of the imaging device 106, based on the distance with respect to the observation target, and thus, it is possible to set the size of the observation target in the captured image captured by the imaging device 106 that performs the pivot motion, to be constant.

(4) Fourth Example of Processing of Control Method

The medical observation apparatus 100 displays the captured image captured by the imaging device 106, on the display device.

Examples of the display device on which the captured image is displayed by the medical observation apparatus 100, include the external display device such as the display device 200. In addition, in a case where the medical observation apparatus 100 includes the display device, the medical observation apparatus 100 may be the display device of the medical observation apparatus 100.

As described above, for example, the medical observation apparatus 100 transmits the display control signal and the image signal subjected to the image processing, to the display device 200 or the like, and thus, displays the captured image on the display device 200 or the like.

[2-1-1] Example of State Transition Realized by Processing of Control Method according to First Embodiment

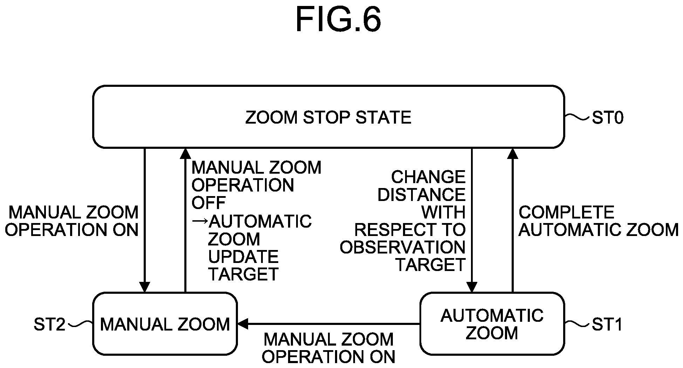

FIG. 6 is an explanatory diagram illustrating an example of a state transition that is realized by the processing of the control method according to the first embodiment, and illustrates an example of a state transition of the zoom control of the imaging device 106 of the medical observation apparatus 100.

Examples of the state of the zoom control of the imaging device 106 include a zoom stop state ST0, automatic zoom ST1, and manual zoom ST2. In addition, as represented in (a) to (c) described below, the medical observation apparatus 100, for example, transitions the state of the zoom control of the imaging device 106.

(a) Transition between Zoom Stop State ST0 and Automatic Zoom ST1

In a case where the distance between the imaging device 106 and the observation target is changed when the state of the zoom control of the imaging device 106 is the zoom stop state ST0, the medical observation apparatus 100 transitions the state of the zoom control of the imaging device 106 to the automatic zoom ST1 from the zoom stop state ST0. In addition, in a case where the automatic zoom is completed, the medical observation apparatus 100 transitions the state of the zoom control of the imaging device 106 to the zoom stop state ST0 from the automatic zoom ST1.

(b) Transition between Zoom Stop State ST0 and Manual Zoom ST2

In a case where the operation relevant to the zoom is detected (in a case where the manual zoom operation is in an on state from an off state) when the state of the zoom control of the imaging device 106 is the zoom stop state ST0, the medical observation apparatus 100 transitions the state of the zoom control of the imaging device 106 to the manual zoom ST2 from zoom stop state ST0. In addition, in a case where the operation relevant to the zoom that is detected, is not detected (in a case where the manual zoom operation is in the off state from the on state), the medical observation apparatus 100 transitions the state of the zoom control of the imaging device 106 to the zoom stop state ST0 from the manual zoom ST2. In addition, in a case where the transition is performed to the zoom stop state ST0 from the manual zoom ST2, the target value is changed to a value corresponding to the operation relevant to the zoom.

(c) Transition to Manual Zoom ST2 from Automatic Zoom ST1

In a case where the operation relevant to the zoom is detected (in a case where the manual zoom operation is in the on state from the off state) when the state of the zoom control of the imaging device 106 is the automatic zoom ST1, the medical observation apparatus 100 transitions the state of the zoom control of the imaging device 106 to the manual zoom ST2 from the automatic zoom ST1.

[2-1-2] Specific Example of Processing of Control Method according to First Embodiment

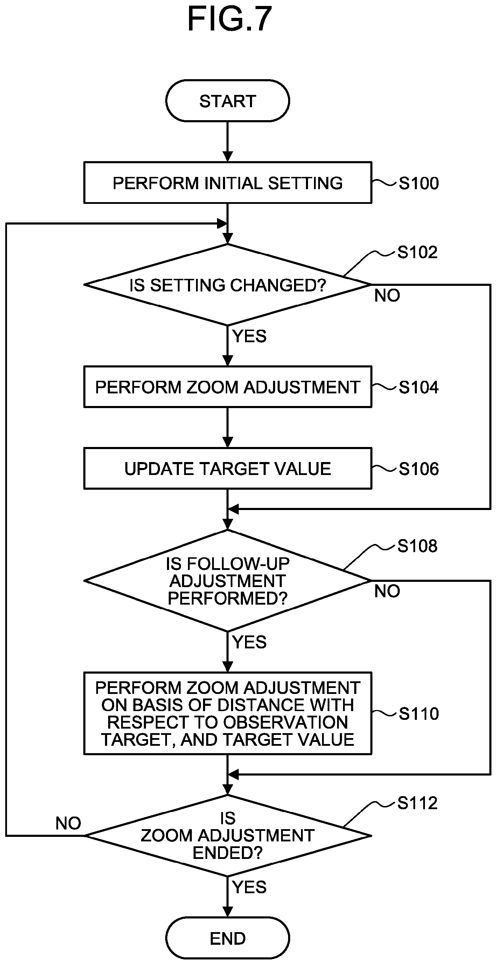

Next, an example of the processing of the control method according to the first embodiment described above, will be described. FIG. 7 is a flowchart illustrating an example of the processing of the control method according to the first embodiment.

The medical observation apparatus 100 performs initial setting (S100). For example, in a case where a main power source of the medical observation apparatus 100 in the on state or in a case where a reset operation of setting the medical observation apparatus 100 to be in an initial state, is detected, the processing of Step S100 is performed.

The medical observation apparatus 100, for example, reads out the information indicating the target value from the recording medium (not illustrated), and thus, performs the initial setting. According to the processing of Step S100, in the medical observation apparatus 100, the target value corresponding to the medical observation apparatus 100 or the target value corresponding to the user of the medical observation apparatus 100 are used as an initial value of the target value.

The medical observation apparatus 100 determines whether or not the setting is changed (S102). For example, in a case where the predetermined operation that is set, is detected, such as a case where the operation relevant to the zoom is detected, or a case where an operation of activating the automatic zoom is detected, the medical observation apparatus 100 determines that the setting is changed.

In a case where it is not determined that the setting is changed in Step S102, the medical observation apparatus 100 performs processing from Step S108 described below.

In addition, in a case where it is determined that the setting is changed in Step S102, the medical observation apparatus 100 performs the zoom adjustment to correspond to the predetermined operation according to Step S102 (S104). As an example of the processing of Step S104, in a case where the operation relevant to the zoom is detected, the medical observation apparatus 100 performs the zoom adjustment to obtains the zoom magnification corresponding to the operation relevant to the zoom. In addition, as another example of the processing of Step S104, in a case where the operation of activating the automatic zoom is detected, the medical observation apparatus 100 performs the zoom adjustment to correspond to the target value that is set. Here, examples of the set target value in Step S104 include a target value indicated by the information indicating the target value read out from the recording medium (not illustrated) in the initial setting of Step S100, or a target value updated by processing of Step S106 described below.

In a case where the zoom adjustment is performed in Step S104, the medical observation apparatus 100 updates the target value to a value corresponding to the state of the zoom after being adjusted (S106). The target value is updated in Step S106, and thus, in the medical observation apparatus 100, the zoom adjustment according to the automatic zoom performed later, is performed based on the target value after being updated.

In a case where it is not determined that the setting is changed in S102, or in a case where the processing of Step S106 is performed, the medical observation apparatus 100 determines whether or not the follow-up adjustment (the automatic zoom) is performed (S108). For example, as represented in processing according to a second example represented in (2) described above, in a case where it is determined that the predetermined operation that is set, is ended, the medical observation apparatus 100 determines that the follow-up adjustment is performed.

In a case where it is not determined that the follow-up adjustment is performed in Step S108, the medical observation apparatus 100 performs processing from Step S112 described below.

In addition, in a case where it is determined that the follow-up adjustment is performed in Step S108, the medical observation apparatus 100 performs the zoom adjustment based on the distance with respect to the observation target and the target value that is set (S110). Here, examples of the set target value in Step S110 include a target value indicated by the information indicating the target value read out from the recording medium (not illustrated) in the initial setting of Step S100, or a target value updated by the processing of Step S106.

In a case where it is not determined that the follow-up adjustment is performed in Step S108, or in a case where the processing of Step S110 is performed, the medical observation apparatus 100 determines whether or not the zoom adjustment is ended (S112). For example, in a case where the predetermined operation that is a trigger for the determination of Step S112, is detected, such as a case where an operation of setting the main power source of the medical observation apparatus 100 to be in the off state is detected, or a case where an operation of fixing the zoom state is detected, the medical observation apparatus 100 determines that the zoom adjustment is ended.

In a case where it is not determined that the zoom adjustment is ended in Step S112, the medical observation apparatus 100 repeats the processing from Step S102.

In addition, in a case where it is determined that the zoom adjustment is ended in Step S112, the medical observation apparatus 100 ends the processing illustrated in FIG. 7.

The medical observation apparatus 100, for example, performs the processing illustrated in FIG. 7, as the processing of the control method according to the first embodiment. Furthermore, it is obvious that the processing of the control method according to the first embodiment is not limited to the example illustrated in FIG. 7.

[2-1-3] Example of Effect Obtained by Using Medical Observation Apparatus according to This Embodiment (or Control Device according to This Embodiment) Performing Processing of Control Method according to First Embodiment

The medical observation apparatus according to this embodiment (or the control device according to this embodiment) performing the processing of the control method according to the first embodiment is used, and thus, for example, the following effects are obtained. Furthermore, it is obvious that the effects obtained by using the medical observation apparatus according to this embodiment (or the control device according to this embodiment) are not limited to the following effects.

- It is not necessary for the surgery operator (an example of the user of the medical observation apparatus according to this embodiment) to manually adjust the zoom after a viewing field is moved according to the movement of the imaging device, and thus, it is possible to smoothly perform the surgery.
- The surgery operator (an example of the user of the medical observation apparatus according to this embodiment) is capable of performing the surgery while peering at the affected area with a constant magnification factor, and thus, it is possible to perform the procedure with an accustomed magnification.

[2-2] Processing of Control Method according to Second Embodiment

As described above, the position of the imaging device 106 can be freely moved, and thus, the position relationship between the imaging device 106 and the observation target becomes fluid.

In addition, the position of the imaging device 106 can be freely moved, and thus, the posture of the imaging device 106 can be changed. Here, in a case where the posture of the imaging device 106 is changed, the direction of the captured image is also changed according to the posture of the imaging device 106. For this reason, in a case where the posture of the imaging device 106 is changed, an aspect ratio of the captured image captured by the imaging device 106 may be considerably changed.

Therefore, the medical observation apparatus 100 further performs processing of adjusting the aspect ratio of the captured image, based on a change in the posture of the imaging device 106, in addition to the processing of the control method according to the first embodiment described above. Hereinafter, in the processing of the control method according to the second embodiment, the description of the same processing as the processing of the control method according to the first embodiment described above will be omitted.

More specifically, the medical observation apparatus 100 adjusts the aspect ratio of the captured image such that the aspect ratio of the captured image approximates to a reference aspect ratio that is set, based on a change in the posture of the imaging device 106. The reference aspect ratio corresponds to a target value of the aspect ratio of the captured image.

The reference aspect ratio according to this embodiment, for example, is set by storing information (data) indicating the reference aspect ratio in the recording medium (not illustrated). In addition, the medical observation apparatus 100, for example, reads out the information indicating the reference aspect ratio from the recording medium (not illustrated), and thus, specifies the reference aspect ratio that is set.

Here, examples of the reference aspect ratio according to this embodiment include an aspect ratio of the captured image captured by the imaging device 106.

Furthermore, the reference aspect ratio according to this embodiment is not limited to the example described above. For example, the reference aspect ratio may be a "value set with respect to the medical observation apparatus 100", a "value associated with one or both of the user of the medical observation apparatus and the surgery method (the procedure) in which the medical observation apparatus 100 is used". In addition, the reference aspect ratio, for example, may be updated at a predetermined timing such as every time when the aspect ratio of the captured image is adjusted.

The medical observation apparatus 100 adjusts the aspect ratio of the captured image such that the aspect ratio of the captured image approximates to the reference aspect ratio, and thus, the aspect ratio of the captured image is identical before the posture of the imaging device 106 is changed and the posture is changed. That is, the medical observation apparatus 100 adjusts the aspect ratio of the captured image such that the aspect ratio of the captured image is identical before the posture of the imaging device 106 is changed and after the posture is changed. Here, "the aspect ratio of the captured image being identical before the posture of the imaging device 106 is changed and after the posture is changed" indicates that "the aspect ratio of the captured image before the change is completely coincident with the aspect ratio after the change" or "a difference in the aspect ratio of the captured image is sufficiently small to the extent that a person peering at the captured image feels that the aspect ratio of the captured image before the change is coincident with the aspect ratio after the change". The upper limit value of a sufficiently small difference in the aspect ratio of the captured image, for example, can be experimentally determined by quantitatively evaluating the result of a "qualitative experiment of allowing a test subject to compare images, and of obtaining a response of whether or not the test subject feels that aspect ratios of the compared images are coincident with each other".

In addition, in a case where the posture of the imaging device 106 is not changed, the medical observation apparatus 100 may not adjust the aspect ratio of the captured image. The medical observation apparatus 100 does not adjust the aspect ratio of the captured image in a case where the posture of the imaging device 106 is not changed, and thus, the load of the processing of the control method according to the second embodiment is reduced. In addition, the load of the processing of the control method according to the second embodiment being reduced, leads to a reduction in the power consumption or a reduction in the heat generation of the medical observation apparatus 100. Furthermore, it is obvious that even in a case where the posture of the imaging device 106 is not changed, the medical observation apparatus 100 may perform processing of adjusting the aspect ratio of the captured image.

The posture of the imaging device 106, for example, is assumed from the state of the arm 104. In addition, the posture of the imaging device 106, for example, may be detected by a gyroscope sensor (not illustrated) or the like, provided in the imaging device 106, the link 112a, or the joint portion 110a.

The medical observation apparatus 100, for example, acquires a projection parameter corresponding to the posture of the imaging device 106, assumed as described above, or a projection parameter corresponding to the posture of the imaging device 106, detected as described above. The medical observation apparatus 100, for example, acquires the projection parameter corresponding to the posture of the imaging device 106, with reference to a "table (or a database) in which the posture of the imaging device 106 and the projection parameter are associated with each other" stored in the recording medium (not illustrated). In addition, the medical observation apparatus 100 may perform an arithmetic operation with respect to an arbitrary algorithm that is capable of calculating the projection parameter based on the posture of the imaging device 106 or the like, and thus, may acquire the projection parameter corresponding to the posture of the imaging device 106.

Then, the medical observation apparatus 100 performs projection conversion using the acquired projection parameter, with respect to the captured image, and thus, adjusts the aspect ratio of the captured image.

Furthermore, the processing of adjusting the aspect ratio of the captured image is not limited to the example described above. For example, the medical observation apparatus 100 is capable of adjusting the posture of the aspect ratio of the captured image according to arbitrary processing that is capable of adjusting the aspect ratio of the captured image based on the imaging device 106.

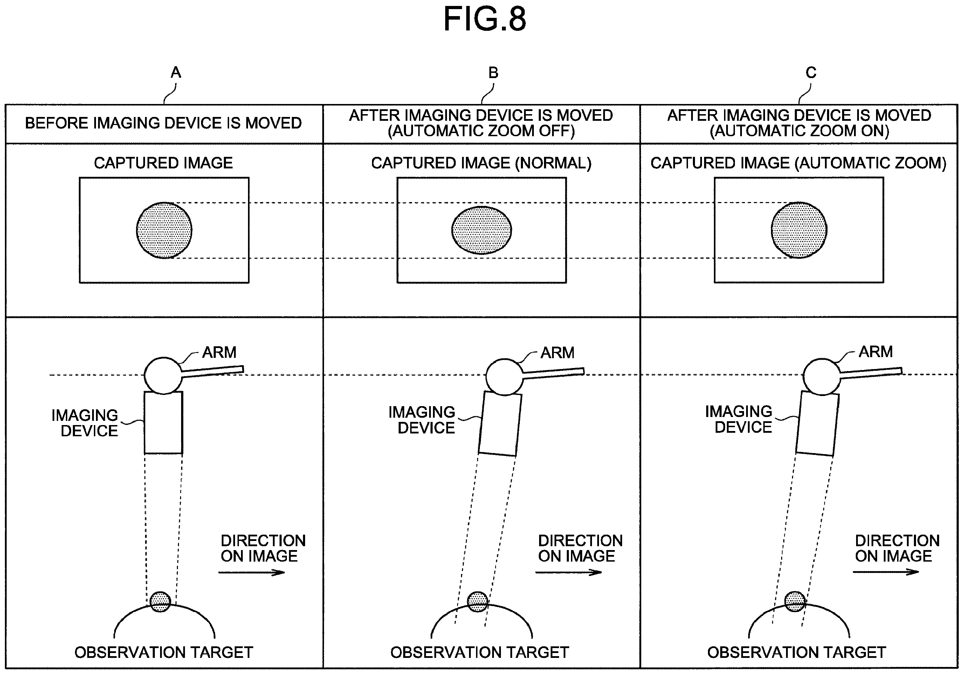

FIG. 8 is an explanatory diagram for describing an example of the processing of the control method according to the second embodiment.

A of FIG. 8 illustrates an example of the captured image before the imaging device 106 is moved.

In addition, B of FIG. 8 and C of FIG. 8 are respectively an example of the captured image after the imaging device 106 is moved, and illustrate an example of the captured image after the distance with respect to the observation target and the posture of the imaging device 106 are changed. More specifically, B of FIG. 8 illustrates an example of the captured image in a case where the processing of the control method according to the second embodiment is not performed. In addition, C of FIG. 8 illustrates an example of the captured image in a case where the processing of the control method according to the second embodiment is performed.

As illustrated in A of FIG. 8, B of FIG. 8, and C of FIG. 8, it is known that in a case where the processing of the control method according to the second embodiment is performed, the size of the observation target in the captured image is identical before the distance is changed and after the distance is changed, and the aspect ratio of the captured image is identical before the posture of the imaging device 106 is changed and after the posture is changed.

Accordingly, the processing of the control method according to the second embodiment is performed, and thus, even in a case where the user of the medical observation apparatus 100 moves the medical observation apparatus 100, it is not necessary to manually adjust the zoom of the imaging device 106, as with a case where the processing of the control method according to the first embodiment is performed. Therefore, the medical observation apparatus 100 performing the processing of the control method according to the second embodiment, is used, and thus, it is possible to improve the convenience.

In addition, the processing of the control method according to the second embodiment is performed, and thus, the aspect ratio of the captured image is identical before the posture of the imaging device 106 is changed and after the posture is changed. That is, in a case where the medical observation apparatus 100 performing the processing of the control method according to the second embodiment, is used, the appearance of the captured image to be displayed on the display screen of the display device 200, becomes constant. Accordingly, the processing of the control method according to the second embodiment is performed, and thus, the surgery operator is capable of performing the surgery while peering at the captured image with constant appearance, and therefore, a change in the state of the affected area is easily grasped. Therefore, the medical observation apparatus 100 performing the processing of the control method according to the second embodiment, is used, and thus, it is possible to improve the convenience.

[2-2-1] Example of State Transition Realized by Processing of Control Method according to Second Embodiment

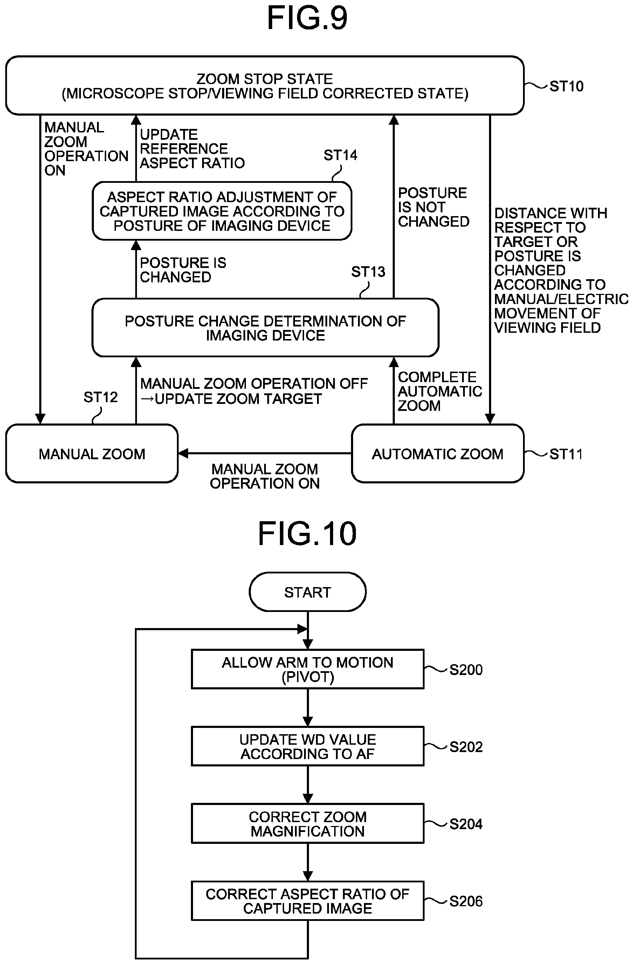

FIG. 9 is an explanatory diagram illustrating an example of a state transition realized by the processing of the control method according to the second embodiment, and illustrates an example of a state transition of the zoom control of the imaging device 106 of the medical observation apparatus 100, and the adjustment of the aspect ratio of the captured image.

Examples of the state of the zoom control of the imaging device 106 include a zoom stop state ST10, automatic zoom ST11, manual zoom ST12, a posture change determination state ST13 of the imaging device 106, and an aspect ratio adjustment state ST14 of the captured image. In addition, as represented in (A) to (F) described below, the medical observation apparatus 100, for example, transitions the state of the zoom control of the imaging device 106.

(A) Transition to Automatic Zoom ST11 from Zoom Stop State ST10

As with the transition between the zoom stop state ST0 and the automatic zoom ST1, illustrated in FIG. 5, the medical observation apparatus 100 transitions the state of the zoom control of the imaging device 106 to the automatic zoom ST11 from the zoom stop state ST10.

(B) Transition to Manual Zoom ST12 from Zoom Stop State ST10

As with the transition between the zoom stop state ST0 and the manual zoom ST2, illustrated in FIG. 5, the medical observation apparatus 100 transitions the state of the zoom control of the imaging device 106 to the manual zoom ST12 from the zoom stop state ST10.

(C) transition to Manual Zoom ST12 from Automatic Zoom ST11

As with the transition to the manual zoom ST2 from the automatic zoom ST1, illustrated in FIG. 5, the medical observation apparatus 100 transitions the state of the zoom control of the imaging device 106 to the manual zoom ST2 from the automatic zoom ST1.

(D) Transition to Posture Change Determination State ST13 from Automatic Zoom ST11/Transition to Posture Change Determination State ST13 from Manual Zoom ST12

In a case where the automatic zoom is completed, the medical observation apparatus 100 transitions the state of the zoom control of the imaging device 106 to the posture change determination state ST13 from the automatic zoom ST11.

In addition, in a case where the operation relevant to the zoom that is detected, is not detected (in a case where the manual zoom operation in the off state from the on state), the medical observation apparatus 100 transitions the state of the zoom control of the imaging device 106 to the posture change determination state ST13 from the manual zoom ST12. In a case where the transition is performed to the posture change determination state ST13 from the manual zoom ST12, the target value is changed to a value corresponding to the operation relevant to the zoom.

(E) Transition to Zoom Stop State ST10 from Posture Change Determination State ST13/Transition to Aspect Ratio Adjustment State ST14 from Posture Change Determination State ST13

In a case where the transition is performed to the posture change determination state ST13, the medical observation apparatus 100 determines whether or not the posture of the imaging device 106 is changed. The medical observation apparatus 100, for example, compares the posture that is previously assumed or detected, with the posture that is currently assumed or detected, and in a case where the compared postures are different from each other, determines that the posture of the imaging device 106 is changed. In addition, in a case where the compared postures are not different from each other, the medical observation apparatus 100 does not determine that the posture of the imaging device 106 is changed.

In a case where it is not determined that the posture of the imaging device 106 is changed, the medical observation apparatus 100 transitions the state of the zoom control of the imaging device 106 to the zoom stop state ST10 from the posture change determination state ST13. In addition, in a case where it is determined that the posture of the imaging device 106 is changed, the medical observation apparatus 100 transitions the state of the zoom control of the imaging device 106 to the aspect ratio adjustment state ST14 from the posture change determination state ST13.

(F) Transition to Zoom Stop State ST10 from Aspect Ratio Adjustment State ST14

In a case where the transition is performed to the aspect ratio adjustment state ST14, the medical observation apparatus 100 performs the processing of adjusting the aspect ratio of the captured image based on the posture that is assumed or detected, and then, performs the transition to the zoom stop state ST10 from the aspect ratio adjustment state ST14.

[2-2-2] Specific Example of Processing of Control Method according to Second Embodiment

Next, an example of the processing of the control method according to the second embodiment described above, will be described. FIG. 10 is a flowchart illustrating an example of the processing of the control method according to the second embodiment, and illustrates an example of processing that is performed in a case where the pivot motion described above is performed.

The medical observation apparatus 100 allows the arm 104 supporting the imaging device 106 to motion such that the pivot motion is realized (S200). In a case where the pivot motion is realized, as described above, the pivot motion is the "motion in which in a state where the imaging device 106 is moved on the plane of the circular cone having the predetermined point as the apex, in the state of being consistently directed towards the predetermined point on the space", and thus, the distance with respect to the observation target and the posture of the imaging device 106 can be changed.

The medical observation apparatus 100 updates a distance WD with respect to the observation target (corresponding to a focus distance) by using the AF function (S202), and corrects the zoom magnification based on the distance WD (S204). The processing of Step S204 corresponds to the processing of the control method according to the first embodiment described above.

In a case where the processing of Step S204 is performed, the medical observation apparatus 100 performs the processing of adjusting the aspect ratio of the captured image, based on the assumed or detected posture of the imaging device 106 (S206). Then, the medical observation apparatus 100 repeats the processing from Step S200.

The medical observation apparatus 100, for example, performs the processing illustrated in FIG. 10, as the processing of the control method according to the second embodiment. For example, the processing illustrated in FIG. 10 is performed, and thus, it is realized that "in a case where the pivot motion is performed, the size of the observation target in the captured image is identical before the distance is changed and after the distance is changed, and the aspect ratio of the captured image is identical before the posture of the imaging device 106 is changed and after the posture is changed". Furthermore, it is obvious that the processing of the control method according to the second embodiment is not limited to the example illustrated in FIG. 10.

[2-2-3] Example of Effect Obtained by Using Medical Observation Apparatus according to This Embodiment (or Control Device according to This Embodiment) Performing Processing of Control Method according to Second Embodiment

The medical observation apparatus according to this embodiment (or the control device according to this embodiment) performing the processing of the control method according to the second embodiment is used, and thus, for example, the following effects are obtained. Furthermore, it is obvious that the effects obtained by using the medical observation apparatus according to this embodiment (or the control device according to this embodiment) are not limited to the following effects.

- The processing of the control method according to the second embodiment includes the processing of the control method according to the first embodiment described above. Accordingly, in a case where the processing of the control method according to the second embodiment is performed, the same effect as that in a case where the processing of the control method according to the first embodiment described above is performed, is obtained.
- After the viewing field is moved, it is not necessary to manually adjust the zoom, it is not necessary for the surgery operator to be accustomed to an angle of the changed viewing field, or it is not necessary to move again the viewing field, and thus, it is possible for the surgery operator to smoothly perform the surgery.
- The stored setting is utilized, and thus, the surgery operator is capable of performing the surgery while peering at constant appearance for each surgery method or each patient, and therefore, a change in the state of the affected area is easily grasped. In addition, it is possible to save a time for the surgery operator to be coordinated with the accustomed appearance, and thus, it is possible to reduce a preparation time of the surgery.

[2-3] Processing of Control Method according to Third Embodiment

As described above, the position of the imaging device 106 can be freely moved, and thus, the position relationship between the imaging device 106 and the observation target becomes fluid. In addition, as illustrated in FIG. 2, there is a case where the imaging device 106 has a "configuration including the first imaging device 130 capturing the first viewing region, and the second imaging device 132 capturing the second viewing region".

Here, the angle of view of the second imaging device 132 is wider than that of the first imaging device 130. Accordingly, in a case where the imaging device 106 has the configuration illustrated in FIG. 2, and the distance with respect to the observation target is changed, a change in the size of the surgical site with respect to the entire captured image or a change in the size of the hands of the surgery operator with respect to the entire captured image, is larger in the captured image of the second imaging device 132 than in the captured image of the first imaging device 130.

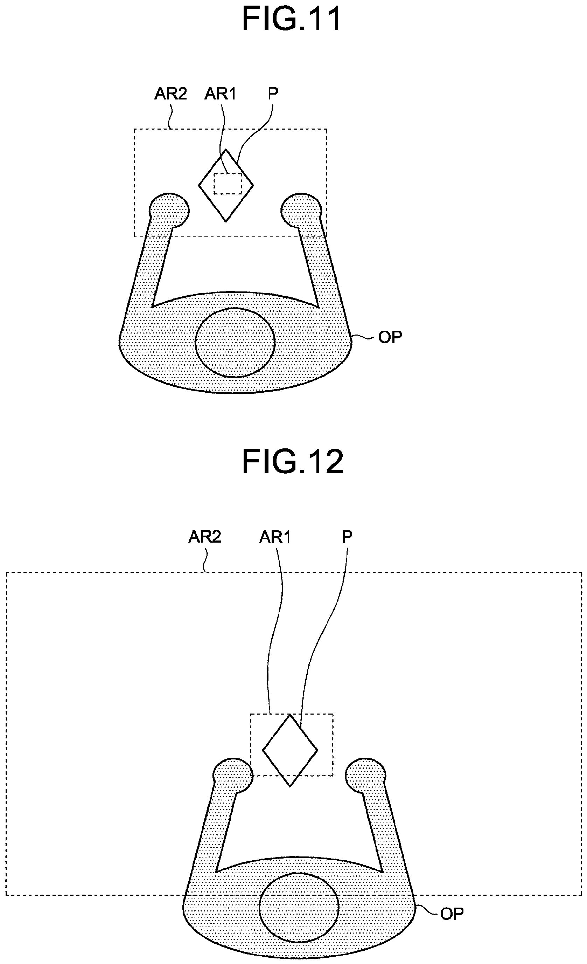

FIG. 11 and FIG. 12 are explanatory diagrams for describing a case where according to processing of a control method according to a third embodiment is not performed. FIG. 12 illustrates a case where the distance with respect to the observation target is farther than that in FIG. 11. "P" illustrated in FIG. 11 and FIG. 12, represents an example of the surgical site that is the observation target, and "OP" illustrated in FIG. 11 and FIG. 12, represents an example of the surgery operator performing a medical practice with respect to the surgical site. "AR1" illustrated in FIG. 11 and FIG. 12, represents an example of the first viewing region of the first imaging device 130, and "AR2" illustrated in FIG. 11 and FIG. 12, represents an example of the second viewing region of the second imaging device 132.

For example, a case where the distance with respect to the observation target is changed to be larger, is assumed. In this case, when the zoom magnification of the imaging device 106 is constant, the first viewing region AR1 of the first imaging device 130 and the second viewing region AR2 of the second imaging device 132 are changed to a state illustrated in FIG. 12 from a state illustrated in FIG. 11. That is, as described above, a change in the size of the surgical site with respect to the entire captured image or a change in the size of the hands of the surgery operator with respect to the entire captured image, is larger in the captured image of the second imaging device 132 than in the captured image of the first imaging device 130.

Accordingly, in a case where the zoom magnification of the imaging device 106 is constant when the distance with respect to the observation target is changed to be larger, there is a "possibility that the surgery operator is not capable of instantly grasping a position relationship between the surgical site and the hands, from the captured image of the second imaging device 132".

Therefore, the medical observation apparatus 100 controls the zoom of the second imaging device 132, based on the distance with respect to the observation target. The distance with respect to the observation target of the control method according to the third embodiment, for example, is measured by the distance sensor 134. That is, the distance with respect to the observation target of the control method according to the third embodiment, can be considered as a distance between the second imaging device 132 and the observation target, and a distance between the first imaging device 130 and the observation target.

As with the processing of the control method according to the first embodiment described above, the medical observation apparatus 100, for example, controls the zoom of the second imaging device 132. That is, the medical observation apparatus 100 controls the zoom such that an index value relevant to the actual field of view of the second imaging device 132 approximates to the target value that is set.

As described above, the second imaging device 132 includes the wide-angle lens or the fish-eye lens. The medical observation apparatus 100 controls a zoom magnification of the wide-angle lens of the second imaging device 132, or a zoom magnification of the fish-eye lens of the second imaging device 132, and thus, the index value relevant to the actual field of view of the second imaging device 132 optically approximates to the target value. Furthermore, the medical observation apparatus 100 is also capable of setting the index value relevant to the actual field of view of the second imaging device 132 as the target value, according to an electronic zoom.

As a specific example, when the distance with respect to the observation target is changed to be 3 times, the medical observation apparatus 100 sets the zoom magnification of the second imaging device 132 to be 3 times. In a case where the distance with respect to the observation target is changed to be 3 times, the dimension of the subject becomes 1/3, but as described above, the zoom magnification of the second imaging device 132 is set to be 3 times, and thus, it is possible to set the second viewing region of the second imaging device 132 not to be changed.

For example, as described above, the medical observation apparatus 100 controls the zoom of the second imaging device 132, and thus, the size of the surgical site with respect to the entire captured image captured by the second imaging device 132, or the size of the hands of the surgery operator with respect to the entire captured image, is identical before the distance with respect to the observation target is changed and after the distance is changed. Accordingly, even in a case where the distance with respect to the observation target is changed, the surgery operator is capable of instantly grasping the position relationship between the surgical site and the hands, from the captured image of the second imaging device 132.

[2-3-1] Specific Example of Processing of Control Method according to Third Embodiment

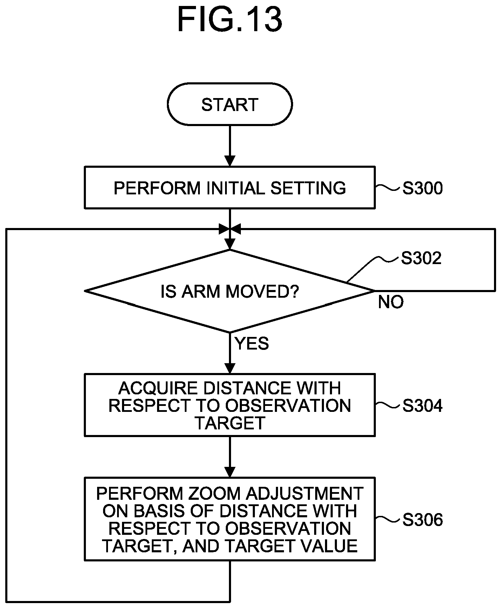

Next, an example of the processing of the control method according to the second embodiment described above, will be described. FIG. 13 is a flowchart illustrating an example of the processing of the control method according to the third embodiment.

As with S100 of FIG. 7, the medical observation apparatus 100 performs the initial setting (S300).

The medical observation apparatus 100 determines whether or not the arm 104 is moved (S302). For example, in a case where the arm 104 is driven, or in a case where the motion mode of the arm 104 is the free mode, the medical observation apparatus 100 determines that the arm 104 is moved. In addition, for example, in a case where the arm 104 is not driven, and the motion mode of the arm 104 is the fixed mode, the medical observation apparatus 100 does not determine that the arm 104 is moved.

In a case where it is not determined that the arm 104 is moved in Step S302, the medical observation apparatus 100 does not proceed with the processing until it is determined that the arm 104 is moved.

In addition, in a case where it is determined that the arm 104 is moved in Step S302, the medical observation apparatus 100 acquires the distance with respect to the observation target (S304). For example, when it is determined that the arm 104 is moved in Step S302, the medical observation apparatus 100 allows the distance sensor 134 to perform measurement, and acquires a detection result of the distance with respect to the observation target from the distance sensor 134. In addition, in the medical observation apparatus 100, the distance sensor 134 may consistently perform the measurement, and when it is determined that the arm 104 is moved in Step S302, the medical observation apparatus 100 may acquire the detection result of the distance with respect to the observation target from the distance sensor 134.

The medical observation apparatus 100 performs the zoom adjustment of the second imaging device 132, based on the distance with respect to the observation target acquired in Step S304 (S306). The medical observation apparatus 100 adjusts the zoom magnification of the second imaging device 132, based on the distance with respect to the observation target, and the target value, and thus, performs the zoom adjustment of the second imaging device 132. Then, the medical observation apparatus 100 repeats the processing from Step S302.

The medical observation apparatus 100, for example, performs processing illustrated in FIG. 13, as the processing of the control method according to the third embodiment. For example, the processing illustrated in FIG. 13 is performed, and thus, it is realized that "the size of the surgical site with respect to the entire captured image captured by the second imaging device 132, or the size of the hands of the surgery operator with respect to the entire captured image, is identical before the distance with respect to the observation target is changed and after the distance is changed". Furthermore, it is obvious that the processing of the control method according to the third embodiment is not limited to the example illustrated in FIG. 13.

[2-3-2] Example of Effect Obtained by Using Medical Observation Apparatus according to This Embodiment (or Control Device according to This Embodiment) Performing Processing of Control Method according to Third Embodiment

The medical observation apparatus according to this embodiment (or the control device according to this embodiment) performing the processing of the control method according to the third embodiment is performed, and thus, for example, the following effects are obtained. Furthermore, it is obvious that the effects obtained by using the medical observation apparatus according to this embodiment (or the control device according to this embodiment) are not limited to the following effects.

- As illustrated in FIG. 2, in a case where the imaging device 106 includes the first imaging device 130 and the second imaging device 132, it is possible to constantly retain the second viewing region of the second imaging device 132, according to the distance with respect to the observation target.
- In a case where the captured image of the second imaging device 132 is displayed on the display area of a part of the display screen on which the captured image of the first imaging device 130 is displayed, the surgery operator is capable of grasping the position relationship between the surgical site and the own hands while keeping eyes on the display screen. Further, the second viewing region of the second imaging device 132 is constantly retained, and thus, even in a case where the distance with respect to the observation target is changed, the surgery operator is capable of easily and instantly grasping the position relationship between the surgical site and the own hands.
- In the processing of the control method according to the third embodiment, the zoom is controlled as with the processing of the control method according to the first embodiment described above, and thus, the same effect as that in a case where the processing of the control method according to the first embodiment described above is performed, is obtained.

[2-4] Processing of Control Method according to Fourth Embodiment

The medical observation apparatus 100 is capable of performing processing in which the processing of the control method according to the first embodiment described above or the processing of the control method according to the second embodiment described above, and the processing of the control method according to the third embodiment described above are combined.

That is, for example, in a case where the imaging device 106 has the configuration illustrated in FIG. 2, the medical observation apparatus 100 is capable of controlling the zoom of each of the first imaging device 130 and the second imaging device 132. In addition, for example, in a case where the imaging device 106 has the configuration illustrated in FIG. 2, the medical observation apparatus 100 is capable of adjusting one or both of an aspect ratio of the captured image of the first imaging device 130 and an aspect ratio of the captured image of the second imaging device 132.

Program according to This Embodiment

A program for allowing a computer system to function as the medical observation apparatus according to this embodiment (or the control device according to this embodiment) (for example, a program capable of executing any one processing of the processing of the control method according to the first embodiment to processing of a control method according to a fourth embodiment), is executed by a processor or the like in the computer system, and thus, it is possible to improve the convenience. Here, examples of the computer system according to this embodiment include a single computer or a plurality of computers. A set of processing of the control method according to this embodiment are performed by the computer system according to this embodiment.

In addition, the program for allowing the computer system to function as the medical observation apparatus according to this embodiment (or the control device according to this embodiment), is executed by the processor or the like in the computer system, and thus, it is possible to obtain the effects obtained by the processing of the control method according to this embodiment described above.

As described above, the preferred embodiments of the present disclosure have been described in detail with reference to the accompanying drawings, but the technical scope of the present disclosure is not limited to such examples. It is apparent that a person with ordinary skill in the technical field of the present disclosure is capable of conceiving various change examples or correction examples within the scope of the technical idea described in the claims, and it is naturally understood that such examples belong to the technical scope of the present disclosure.

For example, in the above description, it is described that the program (a computer program) for allowing the computer system to function as the medical observation apparatus according to this embodiment(or the control device according to this embodiment), is provided, but in this embodiment, a recording medium storing the program described above, can also be provided together.

The configuration described above is an example of this embodiment, and naturally belongs to the technical scope of the present disclosure.

In addition, the effects described herein are merely descriptive or exemplary, and are not limited. That is, in the technology according to the present disclosure, other effects obvious for a person skilled in the art from the description herein, can be obtained along with the effects described above, or instead of the effects described above.

Furthermore, the following configurations also belong to technical scope of the present disclosure.

(1) A medical observation apparatus, including: a control unit that controls a zoom of an imaging device based on a distance between the imaging device supported by an arm in which a plurality of links are connected to each other through a joint portion, and an observation target,

in which the control unit controls the zoom such that an index value relevant to an actual field of view of the imaging device approximates to a target value that is set.

(2) The medical observation apparatus according to (1), in which the control unit controls the zoom such that a size of the observation target in a captured image captured by the imaging device is identical before the distance is changed and after the distance is changed.

(3) The medical observation apparatus according to (2), in which the control unit controls the zoom such that the index value relevant to the actual field of view of the imaging device is identical before the distance is changed and after the distance is changed.

(4) The medical observation apparatus according to any one of (1) to (3), in which the control unit controls the zoom such that the index value relevant to the actual field of view of the imaging device is identical to the target value.

(5) The medical observation apparatus according to any one of (1) to (4), in which the target value is a value set with respect to the medical observation apparatus.

(6) The medical observation apparatus according to any one of (1) to (4), in which the target value is a value associated with one or both of a user of the medical observation apparatus and a surgery method in which the medical observation apparatus is used.

(7) The medical observation apparatus according to any one of (1) to (6), in which the target value is changed based on an operation relevant to the zoom.

(8) The medical observation apparatus according to (7), in which the control unit records the target value that is changed, in a recording medium.

(9) The medical observation apparatus according to any one of (1) to (8), in which the operation relevant to the zoom is detected, the control unit controls the zoom such that the zoom corresponds to the operation relevant to the zoom.

(10) The medical observation apparatus according to (9), in which in a case where the operation relevant to the zoom is detected at the time of controlling the zoom based on the distance, the control unit stops the control of the zoom based on the distance.

(11) The medical observation apparatus according to any one of (1) to (10), in which in a case where it is determined that a predetermined operation that is set, is ended, the control unit controls the zoom based on the distance.

(12) The medical observation apparatus according to any one of (1) to (11), in which the control unit controls the zoom by controlling a zoom magnification of the imaging device based on the distance.

(13) The medical observation apparatus according to (12), in which the control unit controls the zoom within a range of an upper limit value of the zoom magnification and a lower limit value of the zoom magnification.

(14) The medical observation apparatus according to any one of (1) to (13), in which the control unit performs filtering with respect to the distance at a plurality of time points after the distance is changed, and controls the zoom based on the distance subjected to the filtering.

(15) The medical observation apparatus according to any one of (1) to (14), in which the distance is detected by a distance sensor.

(16) The medical observation apparatus according to any one of (1) to (15), in which the control unit displays the captured image captured by the imaging device on a display device.

(17) The medical observation apparatus according to any one of (1) to (16), further including:

the arm; and
the imaging device supported by the arm.

(18) The medical observation apparatus according to (17), in which the distance sensor detecting the distance is provided in the imaging device.

(19) The medical observation apparatus according to any one of (1) to (18), in which the control unit adjusts an aspect ratio of the captured image captured by the imaging device such that the aspect ratio of the captured image approximates to a reference aspect ratio that is set, based on a change in a posture of the imaging device.

(20) The medical observation apparatus according to (19), in which the control unit adjusts the aspect ratio of the captured image such that the aspect ratio of the captured image is identical before the posture is changed and after the posture is changed.

(21) The medical observation apparatus according to (19) or (20), in which in a case where the posture of the imaging device is not changed, the control unit does not adjust the aspect ratio of the captured image.

(22) The medical observation apparatus according to any one of (1), (3) to (8), and (12) to (15), in which the arm supports a first imaging device that captures an image of the observation target, and a second imaging device having an angle of view wider than that of the first imaging device, and the control unit controls a zoom of the second imaging device based on a distance between the second imaging device and the observation target.

(23) The medical observation apparatus according to (22), in which the second imaging device is an imaging device that captures an image of a region including observation target and a circumference of the observation target.

(24) The medical observation apparatus according to (22) or (23), in which the second imaging device includes a wide-angle lens or a fish-eye lens, and the control unit controls a zoom magnification of the wide-angle lens or a zoom magnification or the fish-eye lens.

(25) The medical observation apparatus according to any one of (22) to (24), in which the control unit simultaneously displays a captured image captured by the first imaging device and a captured image captured by the second imaging device, on a display screen of the same display device.

(26) The medical observation apparatus according to any one of (22) to (24), in which the control unit respectively displays a captured image captured by the first imaging device and a captured image captured by the second imaging device, on display screens of different display devices.

(27) The medical observation apparatus according to any one of (22) to (26), further including:

the arm; and
the first imaging device and the second imaging device, supported by the arm.

(29) A control method executed by a medical observation apparatus, the method including:

a step of controlling a zoom of an imaging device based on a distance between the imaging device supported by an arm in which a plurality of links are connected to each other through a joint portion, and an observation target, in which in the step of controlling the zoom, the zoom is controlled such that an index value relevant to an actual field of view of the imaging device approximates to a target value that is set.

Reference Signs List

| | |
|---|---|
| 100 | MEDICAL OBSERVATION APPARATUS |
| 102 | BASE |
| 104 | ARM |
| 106, 136a, 136b | IMAGING DEVICE |
| 110a, 110b, 110c, 110d, 110e, 110f | JOINT PORTION |
| 112a, 112b, 112c, 112d | LINK |
| 130 | FIRST IMAGING DEVICE |
| 132 | SECOND IMAGING DEVICE |
| 134 | DISTANCE SENSOR |
| 152 | ARM PORTION |
| 154 | IMAGING UNIT |
| 156 | COMMUNICATION UNIT |
| 158 | CONTROL UNIT |
| 200 | DISPLAY DEVICE |
| 1000 | MEDICAL OBSERVATION SYSTEM |

**Claims**

1. A medical observation apparatus, comprising
a control unit that controls a zoom of an imaging device based on a distance between: the imaging device supported by an arm in which a plurality of links are connected to each other through a joint portion; and an observation target,

wherein the control unit controls the zoom such that an index value relevant to an actual field of view of the imaging device approximates to a target value that has been set.

2. The medical observation apparatus according to claim 1, wherein the control unit controls the zoom such that a size of the observation target in a captured image captured by the imaging device is identical before the distance is changed and after the distance is changed.

3. The medical observation apparatus according to claim 2, wherein the control unit controls the zoom such that the index value relevant to the actual field of view of the imaging device is identical before the distance is changed and after the distance is changed.

4. The medical observation apparatus according to claim 1, wherein the control unit controls the zoom such that the index value relevant to the actual field of view of the imaging device is identical to the target value.

5. The medical observation apparatus according to claim 1, wherein the target value is a value set with respect to the medical observation apparatus.

6. The medical observation apparatus according to claim 1, wherein the target value is a value associated with one or both of a user of the medical observation apparatus and a surgery method in which the medical observation apparatus is used.

7. The medical observation apparatus according to claim 1, wherein the target value is changed based on an operation relevant to the zoom.

8. The medical observation apparatus according to claim 7, wherein the control unit records the target value that has been changed, in a recording medium.

9. The medical observation apparatus according to claim 1, wherein in a case where an operation relevant to the zoom is detected, the control unit controls the zoom such that the zoom corresponds to the operation relevant to the zoom.

10. The medical observation apparatus according to claim 9, wherein in a case where the operation relevant to the zoom is detected at the time of controlling the zoom based on the distance, the control unit stops the control of the zoom based on the distance.

11. The medical observation apparatus according to claim 1, wherein the control unit controls the zoom based on the distance when it is determined that a predetermined operation that has been set is ended.

12. The medical observation apparatus according to claim 1, wherein the control unit,
performs filtering with respect to the distance at a plurality of time points after the distance is changed, and
controls the zoom based on the distance subjected to the filtering.

13. The medical observation apparatus according to claim 1, wherein the control unit adjusts an aspect ratio of a captured image captured by the imaging device such that the aspect ratio of the captured image approximates to a reference aspect ratio that is set, based on a change in a posture of the imaging device.

14. The medical observation apparatus according to claim 13, wherein the control unit adjusts the aspect ratio of the captured image such that the aspect ratio of the captured image is identical before the posture is changed and after the posture is changed.

15. The medical observation apparatus according to claim 13, wherein in a case where the posture of the imaging device is not changed, the control unit does not adjust the aspect ratio of the captured image.

16. The medical observation apparatus according to claim 1, wherein the arm supports a first imaging device that captures an image of the observation target, and a second imaging device having an angle of view wider than that of the first imaging device, and
the control unit controls a zoom of the second imaging device based on a distance between the second imaging device and the observation target.

**17.** The medical observation apparatus according to claim 16, wherein the second imaging device is an imaging device that captures an image of a region including observation target and a circumference of the observation target.

**18.** The medical observation apparatus according to claim 16, wherein the second imaging device includes a wide-angle lens or a fish-eye lens, and
the control unit controls a zoom magnification of the wide-angle lens or a zoom magnification or the fish-eye lens.

**19.** The medical observation apparatus according to claim 16, wherein the control unit displays a captured image captured by the first imaging device and a captured image captured by the second imaging device, simultaneously on a display screen of the same display device, or respectively on display screens of different display devices.

**20.** A control method executed by a medical observation apparatus, the method comprising
a step of controlling a zoom of an imaging device based on a distance between: the imaging device supported by an arm in which a plurality of links are connected to each other through a joint portion; and an observation target, wherein in the step of controlling the zoom, the zoom is controlled such that an index value relevant to an actual field of view of the imaging device approximates to a target value that has been set.

FIG.1

1000

100

104

106

DISPLAY DEVICE
200

O1, O2, O3, O4, O5, O6

102

110a, 110b, 110c, 110d, 110e, 110f

112a, 112b, 112c, 112d

120, 122, 124, 126, 128

# FIG.2

106

136a    134    136b

130

132

# FIG.3

100

MEDICAL OBSERVATION APPARATUS

152
ARM PORTION

158
CONTROL UNIT

154
IMAGING UNIT

160
IMAGING CONTROLLER

164
DISPLAY CONTROLLER

156
COMMUNICATION UNIT

162
ARM CONTROLLER

# FIG.4

# FIG.5

| A | B | C |
|---|---|---|
| BEFORE IMAGING DEVICE IS MOVED | AFTER IMAGING DEVICE IS MOVED (AUTOMATIC ZOOM OFF) | AFTER IMAGING DEVICE IS MOVED (AUTOMATIC ZOOM ON) |
| CAPTURED IMAGE | CAPTURED IMAGE (NORMAL) | CAPTURED IMAGE (AUTOMATIC ZOOM) |

ARM

IMAGING DEVICE

OBSERVATION TARGET

ARM

IMAGING DEVICE

OBSERVATION TARGET

ARM

IMAGING DEVICE

OBSERVATION TARGET

EP 3 575 847 A1

# FIG.6

ZOOM STOP STATE — ST0

MANUAL ZOOM OPERATION ON

MANUAL ZOOM OPERATION OFF →AUTOMATIC ZOOM UPDATE TARGET

CHANGE DISTANCE WITH RESPECT TO OBSERVATION TARGET

COMPLETE AUTOMATIC ZOOM

ST2 — MANUAL ZOOM

AUTOMATIC ZOOM — ST1

MANUAL ZOOM OPERATION ON

# FIG.7

```
        ┌──────────┐
        │  START   │
        └──────────┘
             │
             ▼
  ┌─────────────────────────┐
  │ PERFORM INITIAL SETTING │ ～S100
  └─────────────────────────┘
             │
             ▼
          ╱─────────╲                 ～S102
         ╱  IS SETTING ╲    NO
        ╲  CHANGED?    ╱ ──────────┐
          ╲─────────╱              │
             │ YES                 │
             ▼                     │
  ┌─────────────────────────┐      │
  │ PERFORM ZOOM ADJUSTMENT │ ～S104│
  └─────────────────────────┘      │
             │                     │
             ▼                     │
  ┌─────────────────────────┐      │
  │   UPDATE TARGET VALUE   │ ～S106│
  └─────────────────────────┘      │
             │                     │
             ▼◄────────────────────┘
          ╱─────────╲                 ～S108
         ╱ IS FOLLOW-UP╲   NO
        ╲  ADJUSTMENT   ╱ ──────────┐
         ╲ PERFORMED?  ╱            │
          ╲─────────╱              │
             │ YES                 │
             ▼                     │
  ┌───────────────────────────┐    │
  │ PERFORM ZOOM ADJUSTMENT   │    │
  │ ON BASIS OF DISTANCE WITH │～S110│
  │ RESPECT TO OBSERVATION    │    │
  │ TARGET, AND TARGET VALUE  │    │
  └───────────────────────────┘    │
             │                     │
             ▼◄────────────────────┘
          ╱─────────╲                 ～S112
   NO    ╱    IS       ╲
 ┌───── ╲ ZOOM ADJUSTMENT╱
 │        ╲   ENDED?    ╱
 │          ╲─────────╱
 │             │ YES
 │             ▼
 │        ┌──────────┐
 │        │   END    │
 │        └──────────┘
 └─────────► (back to S102)
```

# FIG.8

| A | B | C |
|---|---|---|
| BEFORE IMAGING DEVICE IS MOVED | AFTER IMAGING DEVICE IS MOVED (AUTOMATIC ZOOM OFF) | AFTER IMAGING DEVICE IS MOVED (AUTOMATIC ZOOM ON) |
| CAPTURED IMAGE | CAPTURED IMAGE (NORMAL) | CAPTURED IMAGE (AUTOMATIC ZOOM) |

ARM

IMAGING DEVICE

DIRECTION ON IMAGE

OBSERVATION TARGET

EP 3 575 847 A1

# FIG.9

ZOOM STOP STATE
(MICROSCOPE STOP/VIEWING FIELD CORRECTED STATE) — ST10

MANUAL ZOOM OPERATION ON

UPDATE REFERENCE ASPECT RATIO

ST14

ASPECT RATIO ADJUSTMENT OF CAPTURED IMAGE ACCORDING TO POSTURE OF IMAGING DEVICE

POSTURE IS NOT CHANGED

DISTANCE WITH RESPECT TO TARGET OR POSTURE IS CHANGED ACCORDING TO MANUAL/ELECTRIC MOVEMENT OF VIEWING FIELD

POSTURE IS CHANGED

ST13

POSTURE CHANGE DETERMINATION OF IMAGING DEVICE

ST12

MANUAL ZOOM OPERATION OFF →UPDATE ZOOM TARGET

COMPLETE AUTOMATIC ZOOM

MANUAL ZOOM

MANUAL ZOOM OPERATION ON

AUTOMATIC ZOOM — ST11

# FIG.10

START

ALLOW ARM TO MOTION (PIVOT) — S200

UPDATE WD VALUE ACCORDING TO AF — S202

CORRECT ZOOM MAGNIFICATION — S204

CORRECT ASPECT RATIO OF CAPTURED IMAGE — S206

# FIG.11

AR2  AR1    P

OP

# FIG.12

AR2    AR1    P

OP

# FIG.13

```
              ┌─────────────┐
              │    START    │
              └──────┬──────┘
                     │
                     ▼
        ┌──────────────────────────┐
        │ PERFORM INITIAL SETTING  │───S300
        └──────────────┬───────────┘
                       │
    ┌──────────────────▶◀─────────────────────────────┐
    │                  │                               │
    │                  ▼                    ───S302     │
    │            ◇─────────────◇                        │
    │           ╱               ╲                       │
    │          ╱  IS ARM MOVED?  ╲──────────────────────┘
    │          ╲                 ╱      NO
    │           ╲               ╱
    │            ◇─────────────◇
    │                  │
    │                 YES
    │                  ▼
    │      ┌────────────────────────┐
    │      │  ACQUIRE DISTANCE WITH │
    │      │  RESPECT TO OBSERVATION│───S304
    │      │        TARGET          │
    │      └───────────┬────────────┘
    │                  │
    │                  ▼
    │      ┌────────────────────────────┐
    │      │ PERFORM ZOOM ADJUSTMENT    │
    │      │ ON BASIS OF DISTANCE WITH  │
    │      │ RESPECT TO OBSERVATION     │───S306
    │      │ TARGET, AND TARGET VALUE   │
    │      └───────────┬────────────────┘
    │                  │
    └──────────────────┘
```

## INTERNATIONAL SEARCH REPORT

| International application No. |
| --- |
| PCT/JP2017/046822 |

**A. CLASSIFICATION OF SUBJECT MATTER**
Int. Cl. G02B21/26(2006.01)i, A61B90/25(2016.01)i, G02B21/00(2006.01)i

According to International Patent Classification (IPC) or to both national classification and IPC

**B. FIELDS SEARCHED**

Minimum documentation searched (classification system followed by classification symbols)
Int. Cl. G02B21/26, A61B90/25, G02B21/00

Documentation searched other than minimum documentation to the extent that such documents are included in the fields searched
Published examined utility model applications of Japan      1922-1996
Published unexamined utility model applications of Japan    1971-2018
Registered utility model specifications of Japan            1996-2018
Published registered utility model applications of Japan    1994-2018

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | JP 2006-223476 A (OLYMPUS CORP.) 31 August 2006, paragraphs [0009], [0018]-[0058], fig. 1, 2 (Family: none) | 1-11, 20<br>12-19 |
| Y | JP 2011-109644 A (GENERAL ELECTRIC CO.) 02 June 2011, paragraph [0031] & US 2011/0090327 A1, paragraph [0037] & DE 102010038164 A1 & CN 102053355 A | 12 |
| Y | JP 2014-507685 A (CONSTITUTION MEDICAL, INC.) 27 March 2014, paragraph [0016] & WO 2012/105966 A1, page 5, lines 17-26 & EP 2671113 A1 & AU 2011357735 A1 & CA 2826372 A1 & CN 103534628 A & KR 10-2014-0045331 A & HK 1190195 A1 | 12 |

☒ Further documents are listed in the continuation of Box C.        ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 27.02.2018 | 13.03.2018 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**INTERNATIONAL SEARCH REPORT**

| International application No. |
| --- |
| PCT/JP2017/046822 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | WO 2014/061227 A1 (SONY CORP.) 24 April 2014, paragraph [0031] & US 2015/0330776 A1, paragraphs [0032], [0033] & EP 2891916 A1 & CN 104737056 A | 12 |
| Y | JP 2002-354331 A (CANON INC.) 06 December 2002, paragraphs [0013]-[0015] (Family: none) | 13-15 |
| Y | JP 2004-363736 A (CASIO COMPUTER CO., LTD.) 24 December 2004, paragraphs [0043]-[0046] & US 2005/0163396 A1, paragraphs [0091]-[0099] & US 2008/0285878 A1 & WO 2004/107735 A1 & EP 1629665 A1 & TW 200503531 A & KR 10-2006-0015327 A & CN 1799252 A | 13-15 |
| Y | JP 2006-136743 A (OLYMPUS CORP.) 01 June 2006, paragraphs [0047]-[0048] (Family: none) | 16-19 |
| Y | JP 2007-160123 A (OLYMPUS CORP.) 28 June 2007, paragraphs [0033]-[0034] (Family: none) | 16-19 |
| Y | JP 2006-320367 A (OLYMPUS MEDICAL SYSTEMS CORP.) 30 November 2006, paragraphs [0047]-[0049] (Family: none) | 16-19 |

Form PCT/ISA/210 (continuation of second sheet) (January 2015)

**REFERENCES CITED IN THE DESCRIPTION**

**Patent documents cited in the description**

- JP 2016179168 A